# EUROPÄISCHE PATENTSCHRIFT

(11) **EP 1 218 401 B1**
(45) Veröffentlichungstag und Bekanntmachung des Hinweises auf die Patenterteilung: **16.06.2004**
(21) Anmeldenummer: 00964126.7
(22) Anmeldetag: 06.09.2000
(51) Int. Cl.: C07J 43/00, A61K 31/58, A61K 31/575, C07J 9/00, C07J 41/00, A61P 1/16

(54) **4-BENZYLAMINOCHINOLINE KONJUGATE MIT GALLENSAEURE UND IHRE HETEROANALOGEN, VERFAHREN ZU IHRER HERSTELLUNG, DIESE VERBINDUNGEN ENTHALTENDE ARZNEIMITTEL UND DEREN ANWENDUNG**
4-BENZYLAMINOQUINOLINE CONJUGATES WITH BILE ACID AND THEIR HETEROANALOGUES, METHODS FOR PRODUCING THE SAME, MEDICAMENTS CONTAINING THESE COMPOUNDS AND THEIR USE
CONJUGUES DE 4-BENZYLAMINOQUINOLINES CONTENANT DE L'ACIDE BILIAIRE ET LEURS HETEROANALOGUES, LEUR PROCEDE DE PRODUCTION, MEDICAMENTS CONTENANT CES COMPOSES ET LEUR UTILISATION

(30) Priorität: 22.09.1999 DE 19945385; 09.06.2000 DE 10028193
(43) Veröffentlichungstag der Anmeldung: 03.07.2002
(73) Patentinhaber: Aventis Pharma Deutschland GmbH, 65929 Frankfurt am Main (DE)
(72) Erfinder: HOFMEISTER, Armin, 55283 Nierstein (DE); FALK, Eugen, 60529 Frankfurt (DE); KLEEMAN, Heinz-Werner, 65474 Bischofsheim (DE); JANSEN, Hans-Willi, 65527 Niedernhausen (DE); BICKEL, Martin, 61348 Bad Homburg (DE)
(86) Internationale Anmeldenummer: PCT/EP2000/008691
(87) Internationale Veröffentlichungsnummer: WO 2001/021642

(56) Entgegenhaltungen:
- EP-A- 0 624 594
- EP-A- 0 676 410
- DATABASE WPI Section Ch, Week 199102 Derwent Publications Ltd., London, GB; Class B01, AN 1991-012370 XP002158630 & JP 02 286619 A (TOKYO TANABE CO), 26. November 1990 (1990-11-26)
- PATENT ABSTRACTS OF JAPAN vol. 1998, no. 04, 31. März 1998 (1998-03-31) & JP 09 315979 A (TOKYO TANABE CO LTD), 9. Dezember 1997 (1997-12-09)
- DATABASE WPI Section Ch, Week 199906 Derwent Publications Ltd., London, GB; Class B05, AN 1999-070307 XP002160078 & WO 98 56757 A (SANKYO CO LTD), 17. Dezember 1998 (1998-12-17)

## Beschreibung

Die Erfindung betrifft substituierte 4-Benzylaminochinoline und ihre Heteroanalogen, sowie deren pharmazeutisch verträgliche Salze und physiologisch funktionelle Derivate.

Die Bildung von Gallensteinen wird neben einer Reihe von Faktoren wesentlich durch die Zusammensetzung der Galle bestimmt, im besonderen durch die Konzentration und das Verhältnis von Cholesterin, Phospholipiden und Gallensalzen. Voraussetzung für die Bildung von Cholesteringallesteinen ist das Vorhandensein einer an Cholesterin übersattigten Galle (Lit. Carey, M. C. and Small, D.M. (1978) The physical chemistry of cholesterol solubility in bile. Relationship to gallstone formation and dissolution in man, J. Clin. Invest. 61: 998-1026).

Gallensteine werden bislang vorwiegend chirurgisch entfernt, so daß ein großer therapeutischer Bedarf zur medikamentösen Gallensteinauflösung und zur Prävention der Gallensteinbildung besteht.

EP-A-624,594 beschreibt Gallensauer-Anthracen Konjugate zur Behandlung von Gallensteinen und EP-A-676,410 beschreibt Gallensauer-Prolin konjugat zur Behandlung von Gallen steinen.

Der Erfindung lag die Aufgabe zugrunde, Verbindungen zur Verfügung zu stellen, die in der Lage sind, die Bildung von Gallensteinen zu verhindern, indem sie die Übersättigung der Galle mit Cholesterin verhindern, oder indem sie die Bildung von Cholesterinkristallen aus übersättigten Gallen verzögern.

Die Erfindung umfaßt Verbindungen der Formel I 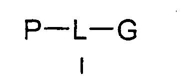 worin bedeuten
- G: 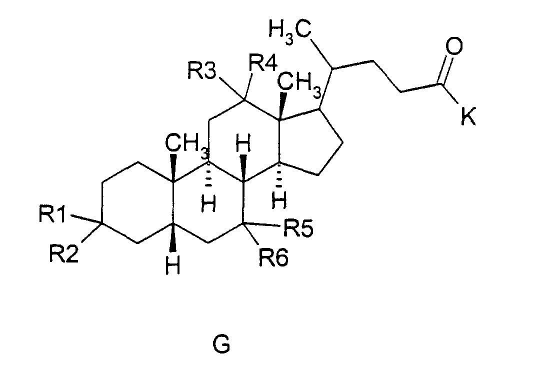
- K: -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -HN-CHR(9)CO₂H, -OKa, wobei Ka ein Kation bedeutet, wie z.B. ein Alkali- oder Erdalkaliion oder ein quartäres Ammoniumion;
- R(7), R(8): unabhängig voneinander Wasserstoff, (C₁-C₄)-Alkyl, Phenyl oder Benzyl, wobei der Phenylkem bis zu 3-fach substituiert sein kann mit F, Cl, CF₃, Methyl, Methoxy;
- R(9): (C₁-C₄)-Alkyl, Benzyl, -CH₂-OH, H₃CSCH₂CH₂-, HO₂CCH₂-, HO₂CCH₂CH₂-;
- R(1 ) bis R(6): unabhängig voneinander Wasserstoff, -OR(10), -SR(10), -NR(10)R(13), -OCOR(10), -SCOR(10), -NHCOR(10),-OPO(OR(10))₂, -OSO₂OR(10), - R(10), R(1 ) und R(2), R(3) und R(4), R(5) und R(6) bilden jeweils gemeinsam den Sauerstoff einer Carbonylgruppe, wobei immer genau einer der Reste R(1 ) bis R(6) die Bedeutung einer Bindung zu L hat;
- R(10), R(13): unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
- L: (C₁-C₁₅)-Alkylen, wobei eine oder mehrere CH₂-Einheiten durch -CH=CH-, -C≡C-, -NR(11)-, -CO-, -O-, -SO₂- oder -S- ersetzt sein können;
- R(11): Wasserstoff, (C₁-C₈)-Alkyl, R(12)-CO-, Phenyl, Benzyl;
- R(12): Wasserstoff, (C₁-C₈)-Alkyl, Phenyl und Benzyl,wobei der Phenylkern bis zu 3-fach substituiert sein kann mit F, Cl, CF₃, Methyl, Methoxy;
- P: 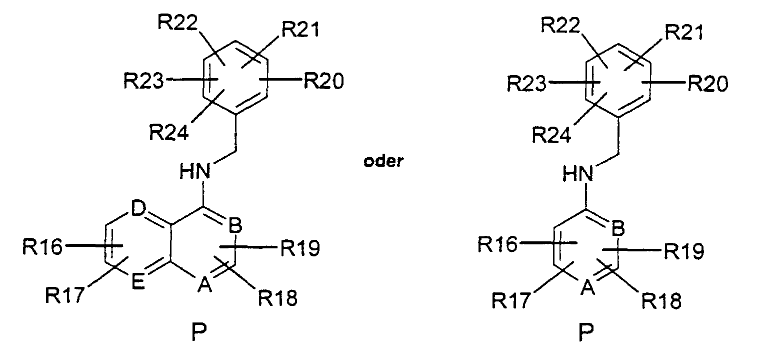
worin bedeuten
- A: N oder CH;
- B: N oder CH;
- D: N oder CH;
- E: N oder CH;
- R(16) bis R(24): unabhängig voneinander Wasserstoff, F, Cl, Br, I, (C₁-C₄)-Alkyl, wobei die Alkylreste ein- oder mehrfach mit Fluor substituiert sein können, CN, NO₂, NR(25)R(26), OR(25), OCOR(25), COR(25), COOR(25), CONR(25)R(26), SO₂R(25), SO₂OR(25), SO₂NR(25)R(26), wobei immer einer der Reste R(16) bis R(24) die Bedeutung einer Bindung zu L hat;
- R(25), R(26): unabhängig voneinander Wasserstoff, (C₁-C₄)-Alkyl, wobei die Alkylreste ein- oder mehrfach mit Fluor substituiert sein können, Phenyl und Benzyl;
sowie deren pharmazeutisch verträglichen Salze und physiologisch funktionelle Derivate.

Bevorzugt sind Verbindungen der Formel I, in denen ein oder mehrere Rest(e) die folgende Bedeutung haben
- G: 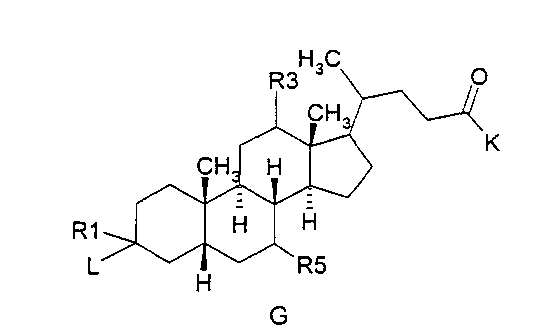
- K: -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -HN-CHR(9)CO₂H, -OKa, wobei Ka ein Kation bedeutet, wie z.B. ein Alkali- oder Erdalkaliion oder ein quartäres Ammoniumion;
- R(7), R(8): unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
- R(9): (C₁-C₄)-Alkyl, Benzyl, -CH₂-OH, H₃CSCH₂CH₂-, HO₂CCH₂-, HO₂CCH₂CH₂-;
- R(1 ), R(3), R(5): unabhängig voneinander Wasserstoff, -OR(10), NR(10) R(13), -OCOR(10), -NHCOR(10);
- R(10), R(13): unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
- L: (C₁-C₈)-Alkylen, wobei eine oder mehrere CH₂-Einheiten durch -CH=CH-, -C≡C-, -NR(11)-, -CO-, -O- oder -SO₂- ersetzt sein können;
- R(11): Wasserstoff, (C₁-C₄)-Alkyl, R(12)-CO-, Phenyl, Benzyl;
- R(12): Wasserstoff, (C₁-C₄)-Alkyl, Phenyl und Benzyl,wobei der Phenylkern bis zu 3-fach substituiert sein kann mit F, Cl, CF₃, Methyl, Methoxy;
- P: 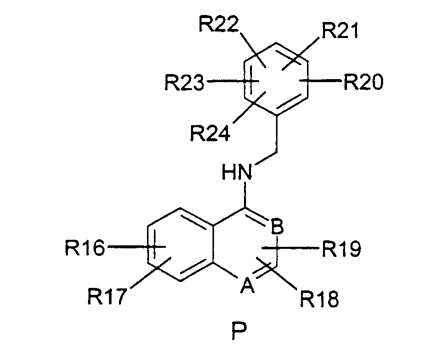
- A: N oder CH;
- B: N oder CH;
- R(16) bis R(24): unabhängig voneinander Wasserstoff, F, Cl, Br, (C₁-C₄)-Alkyl, wobei die Alkylreste ein- oder mehrfach mit Fluor substituiert sein können, NR(25)R(26), OR(25), OCOR(25), COR(25), COOR(25), CONR(25)R(26), wobei immer einer der Reste R(16) bis R(24) die Bedeutung einer Bindung zu L hat;
- R(25), R(26): unabhängig voneinander Wasserstoff, (C₁-C₄)-Alkyl, wobei die Alkylreste ein- oder mehrfach mit Fluor substituiert sein können, Phenyl, Benzyl;
sowie deren pharmazeutisch verträglichen Salze und physiologisch funktionelle Derivate.

Besonders bevorzugt sind Verbindungen der Formel I, in denen ein oder mehrere Reste(e) die folgende Bedeutung hat bzw. haben
- G: 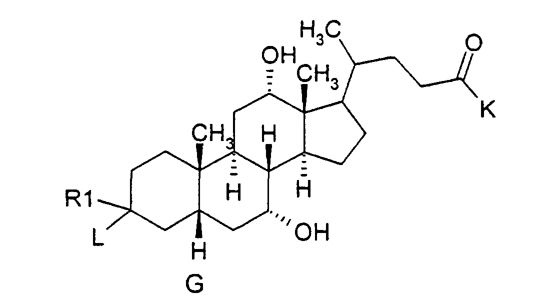
- K: -OR(7), -NR(7) R(8), -HN-CH₂-CH₂-CO₂H, -HN-CH₂-CH₂-SO₃H, -NH-CH₂-CO₂H, -N(CH₃)CH₂CO₂H, -OKa, wobei Ka ein Kation bedeutet, wie z.B. ein Alkali- oder Erdalkaliion oder ein quartäres Ammoniumion;
- R(7), R(8): unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
- R(1): Wasserstoff, -OH;
- L: (C₁-C₅)-Alkylen, wobei eine oder mehrere CH₂-Einheiten durch -CH=CH-, -C≡C-, -NR(11)-, -CO-, -O- oder -SO₂- ersetzt sein können;
- R(11): Wasserstoff, (C₁-C₄)-Alkyl, R(12)-CO-, Phenyl, Benzyl;
- R(12): Wasserstoff, (C₁-C₄)-Alkyl, Phenyl und Benzyl,wobei der Phenylkern bis zu 3-fach substituiert sein kann mit F, Cl, CF₃, Methyl, Methoxy;
- P: 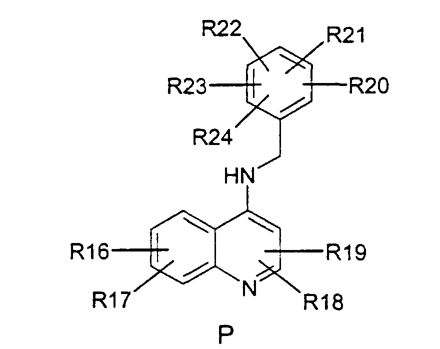
- R(16) bis R(24): unabhängig voneinander Wasserstoff, F, Cl, (C₁-C₄)-Alkyl, wobei die Alkylreste ein- oder mehrfach mit Fluor substituiert sein können, NR(25)R(26), OR(25), OCOR(25), COR(25), COOR(25), CONR(25)R(26), wobei immer einer der Reste R(16) bis R(24) die Bedeutung einer Bindung zu L hat;
- R(25), R(26): unabhängig voneinander Wasserstoff, (C₁-C₄)-Alkyl, wobei die Alkylreste ein- oder mehrfach mit Fluor substituiert sein können, Phenyl und Benzyl;
sowie deren pharmazeutisch verträglichen Salze.

Ganz besonders bevorzugt sind Verbindungen der Formel I, in denen ein oder mehrere Reste die folgende Bedeutung hat bzw. haben
- G: 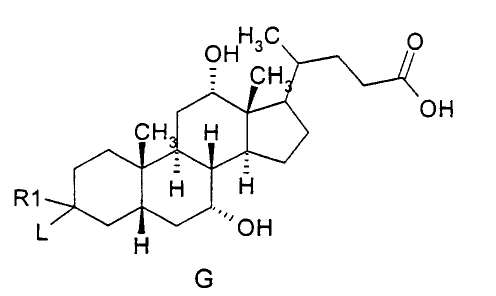
- R(1): Wasserstoff, -OH;
- L: (C₁-C₅)-Alkylen, wobei eine oder mehrere CH₂-Einheiten durch -CH=CH-, -C≡C-, -NR(11 )-, -CO-, -O- oder -SO₂- ersetzt sein können;
- P: 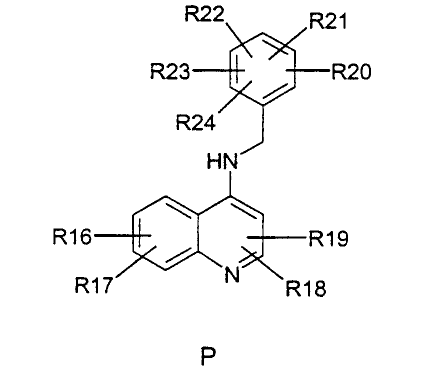
worin bedeutet
- R(16) bis R(24): unabhängig voneinander Wasserstoff, F, Cl, (C₁-C₄)-Alkyl, wobei die Alkylreste ein- oder mehrfach mit Fluor substituiert sein können, NR(25)R(26), OR(25), OCOR(25), COR(25), COOR(25), CONR(25)R(26), wobei immer einer der Reste R(16) bis R(24) die Bedeutung einer Bindung zu L hat;
- R(25), R(26): unabhängig voneinander Wasserstoff, (C₁-C₄)-Alkyl, wobei die Alkylreste ein- oder mehrfach mit Fluor substituiert sein können, Phenyl und Benzyl;
sowie deren pharmazeutisch verträglichen Salze.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Der Ausdruck "wobei der Alkylrest ein oder mehrfach mit F substituiert sein kann" umfaßt auch perfluorierte Alkylreste.

Die bezeichneten Alkylreste können sowohl geradkettig wie verzweigt vorliegen.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-,Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chloridsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.
Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten.

Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des Salzes der Verbindung der Formel I. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säureund magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Die Erfindung betrifft weiterhin zwei Verfahren zur Herstellung der Verbindungen der Formel I.

Verfahren A) Dabei wird in einer Pd(0)-katalysierten Kupplungsreaktion eine Verbindung der Formel IIId, bei der X Br oder I bedeutet mit IId zur Reaktion gebracht. Das dabei frei werdende HX wird mittels eine Hilfsbase (wie z.B. Triethylamin oder Pyridin) abgefangen. 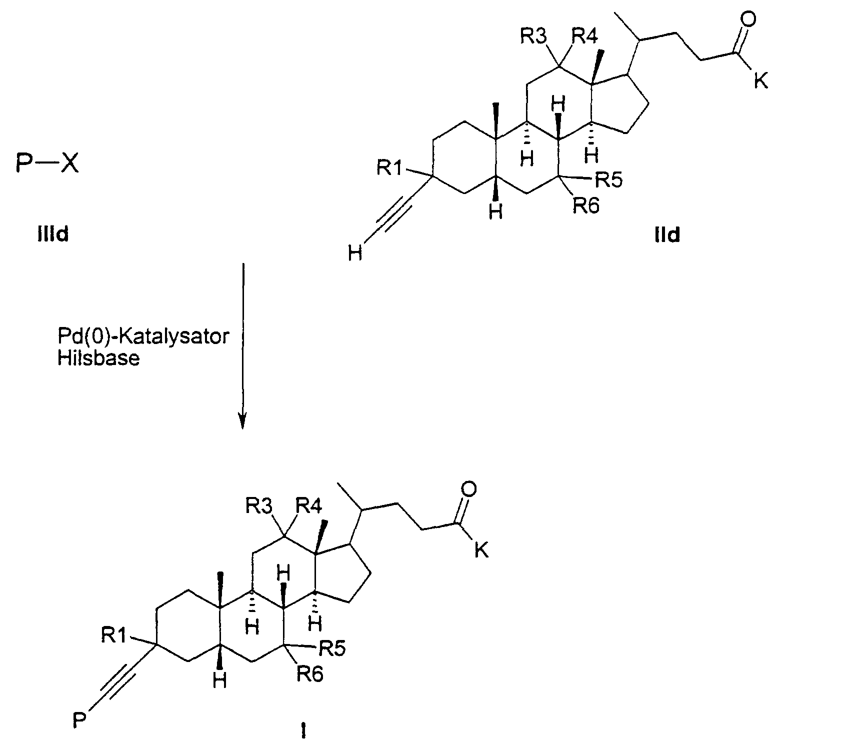 R1, R2, R3, R4, R5, K und P haben die oben genannte Bedeutung. Die Acetylen-Gallensäurederivate der Formel IId werden aus geeigneten Gallensäureketonen hergestellt. Dazu wird Lithiumacetylid analog zu bekannten Verfahren (US 5, 641, 767) an Ketogallensäuren addiert.

Verfahren B) Carbonsäuren der Formel IIIe (R = OH) werden in Gegenwart geeigneter Kupplungsreagenzien wie z.B. TOTU (Chemiker Zeitung, 98 (1974), 817), DCC/HOBt (J. Am. Chem. Soc., 77 (1955), 1067) oder CMC/HOBt (J. Org. Chem., 21 (1956), 439), (s. Abkürzungen) mit Verbindungen der Formel IIe in bekannter Weise umgesetzt, wodurch sich eine Amidbindung bildet. Ebenso lassen sich die gezeigten Carbonsäureamide I durch Umsetzung von aktivierten Carbonsäurederivaten IIIe mit Verbindungen der Formel IIe in Gegenwart einer Hilfsbase (z. B. Triethylamin oder Pyridin) in dem Fachmann bekannter Weise herstellen. Als aktivierte Carbonsäurederivate sind hier z. B. die entsprechenden Chloride (R = Cl), Imidazolide (R = 1-Imidazolyl; Angew. Chem. Int. Ed. Engl.,1 (1962), 351) oder die gemischten Anhydride mit Cl-COOEt oder Tosylchlorid zu nennen. 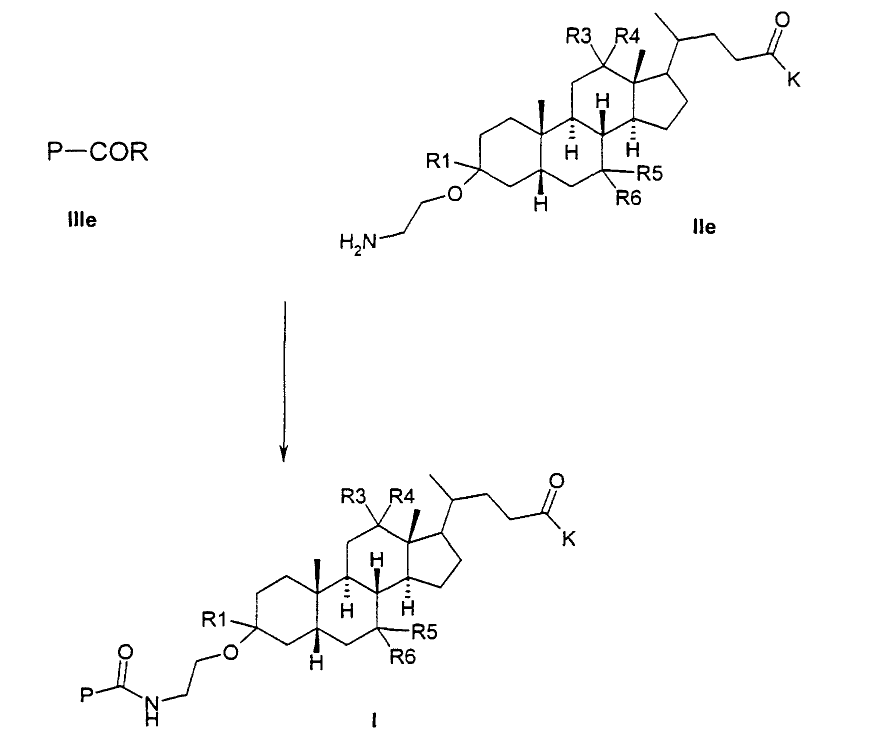 R1, R2, R3, R4, R5, K und P haben die oben genannte Bedeutung. Die 3-Ethanolamin-Gallensäurebausteine der Formel IIe werden dabei nach bekannten Verfahren (Tetrahedron Lett., 34 (1993), 817) hergestellt.

Die Verbindungen der Formel I und deren pharmazeutisch verträgliche Salze und physiologisch funktionelle Derivate zeichnen sich durch eine günstige Beeinflussung der Gallezusammensetzung aus und verhindern die Bildung von Gallensteinen, indem sie die Übersättigung der Galle mit Cholesterin verhindern, oder indem sie die Bildung von Cholesterinkristallen aus übersättigten Gallen verzögern. Die Verbindungen können allein oder in Kombination mit lipidsenkenden Wirkstoffen (siehe Rote Liste, Kapitel 58) eingesetzt werden. Die Verbindungen eignen sich insbesondere zur Prophylaxe sowie zur Behandlung von Gallensteinen.

Die erfindungsgemäßen Verbindungen der Formel (I) gelangen in das hepatobiliäre System und wirken daher in diesen Geweben. So wird die Wasserabsorption aus der Gallenblase durch Inhibition des apikalen NHE-Antiports vom Subtyp 3 des Gallenblasenepithels gehemmt, was eine verdünnte Gallenflüssigkeit zur Folge hat.

Die biologische Prüfung der erfindungsgemäßen Verbindungen erfolgte durch Ermittlung der Inhibition des Natrium / Protonen-Austauscher Subtyp 3.

### 1. Testbeschreibung

Zur Bestimmung der Restaktivität von humanem NHE-3 Protein (exprimiert in LAP1-Zellinie) wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl-freien Puffers (s. u.) zu 25 µl Aliquots der in NH₄Cl-Puffer inkubierten Zellen induziert. Die nachfolgende Geschwindigkeit der pH-Erholung wurde 3 Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pHs bei der getesteten Konzentration einer Substanz wurde in Prozent der maximalen Erholung ausgedrückt.

### Ergebnisse:

- Beispiel 1:: Restaktivität des hNHE3 bei 30 µM = 26 %
- Beispiel 2:: Restaktivität des hNHE3 bei 30 µM = 33 %
- Beispiel 3:: Restaktivität des hNHE3 bei 30 µM = 22 %
- Beispiel 6:: Restaktivität des hNHE3 bei 30 µM = 39 %
- Beispiel 11:: Restaktivität des hNHE3 bei 30 µM = 61 %
- Beispiel 13:: Restaktivität des hNHE3 bei 30 µM = 40 %
- Beispiel 14:: Restaktivität des hNHE3 bei 30 µM = 52 %
- Beispiel 15:: Restaktivität des hNHE3 bei 30 µM = 65 %
- Beispiel 16:: Restaktivität des hNHE3 bei 30 µM = 27 %
- Beispiel 17:: Restaktivität des hNHE3 bei 30 µM = 36 %
- Beispiel 18:: Restaktivität des hNHE3 bei 30 µM = 36 %
- Beispiel 20:: Restaktivität des hNHE3 bei 30 µM = 56 %
- Beispiel 23:: Restaktivität des hNHE3 bei 30 µM = 16 %
- Beispiel 24:: Restaktivität des hNHE3 bei 30 µM = 29 %
- Beispiel 25:: Restaktivität des hNHE3 bei 30 µM = 18 %
- Beispiel 26:: Restaktivität des hNHE3 bei 30 µM = 52 %
- Beispiel 27:: Restaktivität des hNHE3 bei 30 µM = 54 %
- Beispiel 28:: Restaktivität des hNHE3 bei 30 µM = 69 %
- Beispiel 29:: Restaktivität des hNHE3 bei 30 µM = 61 %
- Beispiel 30:: Restaktivität des hNHE3 bei 30 µM = 48 %
- Beispiel 31:: Restaktivität des hNHE3 bei 30 µM = 69 %

### Liste der Abkürzungen:

- Me: Methyl
- LAH: Lithiumaluminiumhydrid
- DMF: N,N-Dimethylformamid
- EI: electron impact
- Cl: Chemical lonisation
- RT: Raumtemperatur
- EE: Ethylacetat
- mp: Schmelzpunkt
- HEP: n-Heptan
- DME: Dimethoxyethan
- ES: Elektronenspray
- FAB: Fast Atom Bombardment
- THF: Tetrahydrofuran
- eq.: Äquivalent
- TOTU: O-[(Ethoxycarbonyl)-cyanmethylenamino]-N,N,N',N'-teramethyluronium-terafluoroborat
- HOBt: 1-Hydroxy-benzotriazol
- CMC: N-Cyclohexy)-N'-(2-morpholinoethyl)-carbodiimid-methyl-p-toluolsulfonat
- DCC: Dicyclohexylcarbodiimid

Die nachfolgenden Beispiele dienen zur näheren Erläuterung der Erfindung, ohne dieselbe auf in den Beispielen beschriebene Produkte und Ausführungsformen einzuschränken.

### Beispiel 1

### 2-Methyl-3-(aceto-1-yl)-4-{4-[1-(3α,7α,12α -trihydroxy-10β,13β -dimethyl-17β-(pentansäure-4-yl)-hexadecahydro-cyclopenta[a]phenanthren-3-yl)-ethin-2-yl]-benzyl}-amino-chinolin

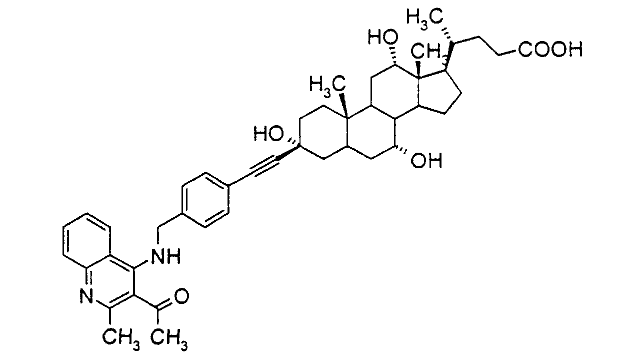

Darstellung der Zwischenprodukte:
Zwischenprodukt 1: 3β-Acetylen-cholsäure

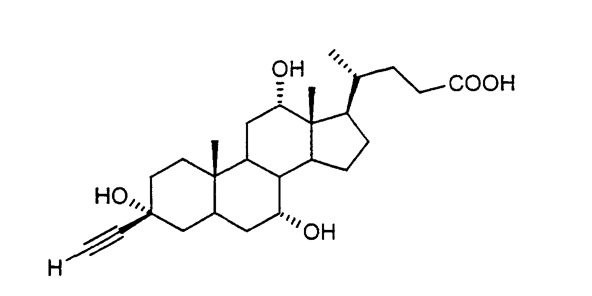

### Syntheseweg:

a) 3,7,12-Triacetylcholsäuremethylester
   90 g Cholsäuremethylester und 3.0 g Dimethylaminopyridin wurden in 500 ml Pyridin gelöst, mit 500 ml Acetanhydrid versetzt und über Nacht bei Zimmertemperatur gerührt. Es wurde auf Eiswasser gegossen und mit Ethylacetat (3x) extrahiert. Trocknen (MgSO₄) und Eindampfen der organischen Phase ergaben 92 g 3,7,12-Triacetylcholsäuremethyl-ester, MS(FAB): M⁺+Li = 555.
b) 7,12-Diacetyl-cholsäuremethylester
   Bei 5°C wurden 150 ml Acetanhydrid langsam in 1.5 l Methanol zugetropft. Nach 15 Minuten wurden 92 g 3,7,12-Triacetylcholsäuremethylester zugegeben und 1 h bei Zimmertemperatur gerührt. Es wurde auf Eiswasser gegossen und mit Ethylacetat (3x) extrahiert. Die organische Phase wurde mit 1 N Na₂CO₃-Lösung gewaschen, mit MgSO₄ getrocknet und eingedampft. Es wurden 85 g Rohprodukt erhalten, MS(FAB): M⁺+Li = 513.
c) 3-Keto-7,12-diacetyl-cholsäuremethylester
   85 g (168 mmol) 7,12-Diacetylcholsäuremethylester, 183.7 g Pyridiniumchlorochromat und 175 g Molekularsieb wurden in 2.5 l Dichlormethan 2 h bei Zimmertemperatur gerührt. Es wurde auf 7 l Diethylether gegossen, die Feststoffe abfiltriert. Das Lösungsmittel wurde eingedampft und der Rückstand in Ethylacetat gelöst. Nach Chromatographie über eine Florisil-Säule wurden 59.6 g Produkt erhalten, MS(FAB): M⁺+Li = 511.
d) 3β-Acetylen-7,12-diacetyl-cholsäuremethylester
   In 750 ml abs. Tetrahydrofuran wurde bei -55°C unter Argon 25 min Acetylen eingeleitet. Zu dieser Lösung wurden 145 ml 15% n-Butyllithium in Hexan zugetropft und 10 min nachgerührt. Anschließend wurden 45 g (89 mmol) 3-Keto-7,12-diacetylcholsäuremethylester zugegeben und 1.5 h bei -40°C gerührt. Zur Aufarbeitung wurden 500 ml gesättigte wäßrige Ammoniumchloridlösung zugegeben und mit Ethylacetat (3x) extrahiert, die organische Phase über MgSO₄ getrocknet und eingedampft. Der Rückstand wurde über Kieselgel chromatographiert (n-Heptan/ Ethylacetat 1:1). Es wurden 35.3 g Produkt erhalten, MS(FAB): M⁺+Li = 537.
e) 3β-Acetylen-cholsäure
   35.2 g (66 mmol) des Produkts aus d) wurden in 1 l Methanol gelöst, mit 300 ml 2N Natriumhydroxidlösung versetzt und 25 h unter Rückfluß erhitzt. Das Lösungsmittel wurde eingedampft, der Rückstand in Wasser gelöst und mit 2N Salzsäure bis pH 2 angesäuert. Der Niederschlag wurde abfiltriert und mit Wasser neutral gewaschen. Trocknung des Rückstandes ergab 14.6 g Produkt, MS(FAB): M⁺+Li = 439.

### Zwischenprodukt 2: 2-Methyl-3-(aceto-1-yl)-4-(4-bromobenzyl)-amino-chinolin

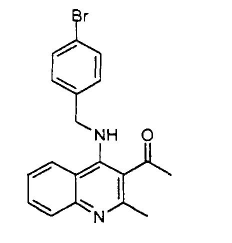

### Syntheseweg

a) 2-(1-Methyl-3-oxo-but-1-enylamino)-benzonitril gemäß Standartmethoden aus 2-Aminobenzonitril hergestellt (Eur. Pat. Appl., C07D 215/42; J. Med. Chem., 31 (1988), 1278); gelber Feststoff; Schmp.: 100°C; MS(Cl): M⁺+H = 201.
b) 1-(4-Amino-2-methyl-chinolin-3-yl)-ethanon kann ausgehend von 2-(1-Methyl-3-oxo-but-1-enylamino)-benzonitril durch Cyclisierung mit CuCl und K₂CO₃ (J. Med. Chem., 31 (1988), 1278) oder NaOMe-vermittelt (Eur. Pat. Appl., C07D 215/42) nach bekanntem Verfahren hergestellt werden; gelblicher Feststoff; MS(Cl): M⁺+H = 201.
c) 1-(4-(4-Bromo-benzylamino-2-methyl-chinolin-3-yl)-ethanon
   In einem Zweiphasensystem aus 75 ml CH₂Cl₂ und 55 ml 50 %ige NaOH werden 1.8 g 1-(4-Amino-2-methyl-chinolin-3-yl)-ethanon mit 0.15 eq Tetrabutylammoniumhydrogensulfat versetzt und 30 min bei Raumtemperatur kräftig gerührt. Danach werden 1.1 eq 4-Brombenzylbromid zugegeben und 4 bis 5 h bei Raumtemperatur kräftig gerührt. Zur Aufarbeitung werden die beiden Phasen getrennt, die wäßrige noch 2 x mit CH₂Cl₂ extrahiert und die vereinigten organischen Phasen noch 2 x mit H₂O gewaschen und mit MgSO₄ getrocknet. Das Lösungsmittel wird abdestilliert und der Rückstand an Kieselgel (300 g; CH₂Cl₂ / MeOH 98:2) gereinigt, wobei die Titelverbindung als farbloser, glasiger Feststoff erhalten wird. MS(ES+): M⁺+H = 369.

### Allgemeine Vorschrift für die Pd(0)-katalysierte Kupplungsreaktion

Die Bromarylverbindung (1.0 eq) und der Gallensäure-acetylenbaustein (1.5 eq) werden in DMF / Triethylamin (2:1) vorgelegt und die Lösung entgast. Nach Belüften mit Argon werden je 0.1 eq Pd(PPh₃)₂Cl₂ und 0.1 eq Cul zugegeben und die Reaktionslösung auf 80 °C erwärmt. Je nach Reaktionsverlauf ist es sinnvoll weiteren Katalysator zuzugeben oder die Temperatur noch zu erhöhen, wobei bis zu 100°C erreicht werden können. Zur Aufarbeitung wird das Lösungsmittel entfernt und das so erhaltene Rohprodukt an Kieselgel mit einer CH₂Cl₂/MeOH-Mischung gereinigt.

### 2-Methyl-3-(aceto-1-yl)-4-{4-[1-(3α,7α,12α-trihydroxy-10β,13β-dimethyl-17β-(pentansäure-4-yl)-hexadecahydro-cyclopenta[a]phenanthren-3-yl)-ethin-2-yl]-benzyl}-amino-chinolin

Reaktionsführung nach der allgemeinen Vorschrift ergibt nach 21 h bei 100°C einen gelb-orangen Feststoff, Schmp.: 178°C (Zers.), MS(ES+): M⁺+H = 772.

### Beispiel 2:

### 2-Methyl-3-(aceto-1-yl)-4-{4-[1-(3α,7α,12α -trihydroxy-10β,13β -dimethyl-17β-(pentansäure-4-yl)-hexadecahydro-cyclopenta[a]phenanthren-3-yl)-eth-2-yl]-benzyl}amino-chinolin

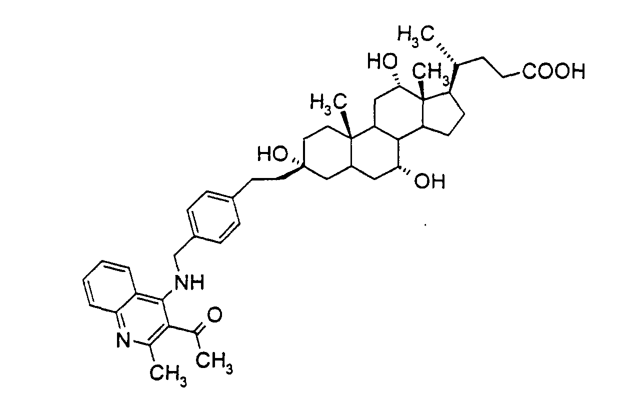

Das oben erhaltene Produkt aus Beispiel 1 wird in Ethanol/Methanol 1:1 gelöst, mit Pd / C (10 %ig) versetzt und unter H₂-Atmosphäre geschüttelt bis vollständiger Umsatz erreicht ist. Abfiltrieren des Pd-Katalysators und Abdestillieren des Lösungsmittels liefert das hydrierte Produkt als gelblichen, kristallinen Feststoff. Schmp.: 172°C (Zers.); MS(ES+): M⁺+H = 726.

### Beispiel 3:

### 2-Methyl-4-aminobenzyl-chinolin-3-carbonsäure-N-{2-O-[7α,12α-dihydroxy-10β,13β-dimethyl-17β-(pentansäuremethylester-4-yl)-hexadecahydrocyclopenta[a]phenanthren-3β-yl]}-ethylamid

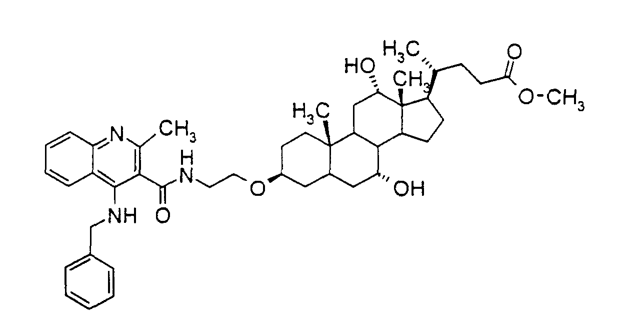

Darstellung der Zwischenprodukte:
Zwischenprodukt 1: 3β-O-(1-amino-et-2-yl)-cholsäuremethylester

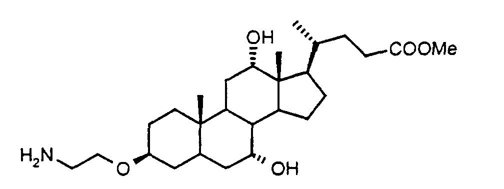

Die Titelverbindung kann in literaturbekannter Weise ausgehend von Cholsäure in sechs Schritten hergestellt werden. (Tetrahedron Lett., 33 (1992), 195; Tetrahedron Lett., 34 (1993), 817).

### Zwischenprodukt 2: 4-Benzylamino-2-methyl-chinolin-3-carbonsäure

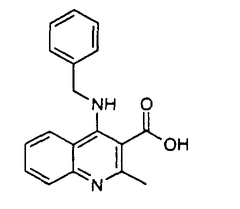

### Syntheseweg:

a) 4-Amino-2-methyl-chinolin-3-carbonsäure-methylester wird nach dem Fachmann bekannter Vorschrift hergestellt (Tetrahedron, 51 (1995), 12277). MS(Cl): M⁺+H = 217.
b) 4-Benzylamino-2-methyl-chinolin-3-carbonsäure-methylester wird nach dem gleichen Verfahren synthetisiert, wie bereits für 1-(4-(4-Bromo-benzylamino-2-methyl-chinolin-3-yl)-ethanon (Beispiel 1, Zwischenprodukt 2c) beschrieben wurde, ausgehend von 4-Amino-2-methyl-chinolin-3-carbonsäure-methylester und Benzylbromid. MS(ES+): M⁺+H = 307.
c) 4-Benzylamino-2-methyl-chinolin-3-carbonsäure wird durch Verseifung des Methylesters mit KOH (5 eq) in ethanolischer Lösung hergestellt. Nach vollständigem Umsatz wird das Lösungsmittel entfernt und das Rohprodukt in 2N NaOH aufgenommen. Die wässrige Lösung wird mit CH₂Cl₂ extrahiert, die Phasen getrennt und die wässrige Phase mit 2N HCl neutralisiert, wobei die Titelverbindung ausfällt. Abfiltrieren und Trocknen liefert einen farblosen Feststoff. Schmp.: 190°C; MS(Cl): M⁺+H = 293, M⁺-CO₂ = 249.

### 2-Methyl-4-aminobenzyl-chinolin-3-carbonsäure-N-{2-O-[7α,12α-dihydroxy-10β,13β-dimethyl-17β-(pentansäuremethylester-4-yl)-hexadecahydrocyclopenta[α]phenanthren-3β-yl]}-ethylamid als Trifluoracetat

Zwischenprodukt 1 (1.0 eq) und Zwischenprodukt 2 (1.0 eq) werden in DMF vorgelegt, 1.0 eq HOBt zugegeben und bei 0°C mit einer Lösung von 1.1 eq CMC in DMF versetzt. Wenn nötig wird zusätzlichen CMC zugegeben und/oder die Temperatur erhöht, wobei 60°C erreicht werden können. Zur Aufarbeitung wird vom Lösungsmittel befreit, der Rückstand in Essigester aufgenommen und 2 x mit ges. NaHCO₃ gewaschen. Die NaHCO₃-Phasen werden noch einmal mit Essigester extrahiert und die vereinigten organischen Extrakte noch 2 x mit H₂O gewaschen, mit MgSO₄ getrocknet und einrotiert. Das so erhaltene Rohprodukt wurde durch präparative HPLC gereinigt, wobei die Titelverbindung als leicht gelblicher Feststoff erhalten wurde. Schmp.: 102°C; MS(ES+): M⁺+H = 741.

### Beispiel 4:

### 2-Methyl-4-aminobenzyl-chinolin-3-carbonsäure-N-{2-O-[7α,12α-dihydroxy-10β,13β-dimethyl-17β-(pentansäure-4-yl)-hexadecahydro-cyclopenta[a]phenanthren-3β-yl]}ethylamid

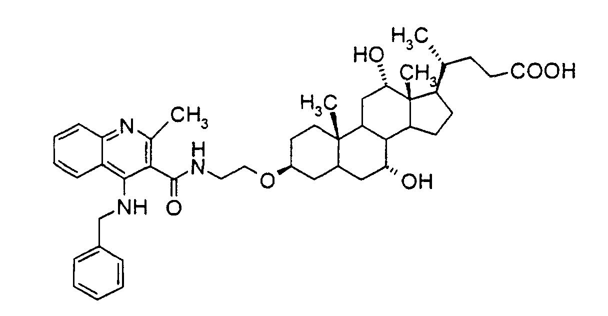

Das Produkt aus Beispiel 3 wird mit 5.0 eq KOH in einem Methanol/H₂O-Gemisch versetzt und bei einer Temperatur zwischen Raumtemperatur und 50°C gerührt. Wenn nötig wird zusätzliches KOH zugesetzt bis vollständiger Umsatz erreicht ist. Danach wird vom Lösungsmittel befreit, der Rückstand in H₂O aufgenommen und mit 1 N HCl neutralisiert. Der entstandene Niederschlag wird abfiltriert und getrocknet, wobei das Produkt als farbloser Feststoff erhalten wird. Schmp.: 143°C;
MS(ES+): M⁺+H = 727.

### Beispiel 5:

### 2-Methyl-4-aminobenzyl-chinolin-3-carbonsäure-N-{2-O-[7α,12α, dihydroxy-10β,13β-dimethyl-17β-(pentansäure-N-(2-sulfonsäure)-ethylamid-4-yl)-hexadecahydrocyclopenta[a]phenanthren-3β-yl]}-ethylamid als Trifluoracetat

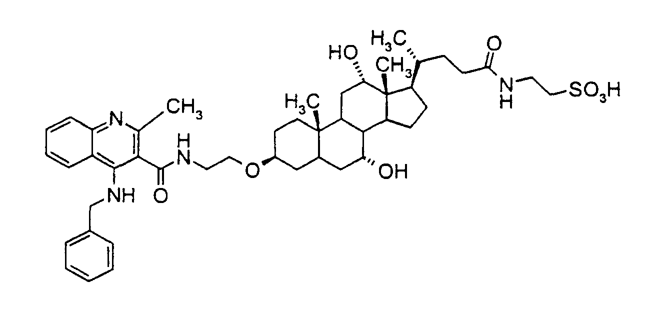

85 mg der Produktsäure aus Beispiel 3 werden in 5 ml abs. DMF gelöst. Bei 0°C wird eine Lösung von 0.016 ml NEt₃ in 1 ml abs. DMF zugegeben und 1.0 eq TOTU, gelöst in 2 ml abs. DMF zugetropft. Nach 1 h Rühren zwischen 0°C und Raumtemperatur wird die so erhaltene Lösung zu einer Mischung aus 1.0 eq Taurin, 1 ml NEt₃, 2 ml H₂O sowie 2 ml DMF gegeben und weiter gerührt. Zur Aufarbeitung wird das Reaktionsgemisch eingeengt und durch präparative HPLC gereinigt, wobei die Titelverbindung als gelblicher Feststoff anfällt. Schmp.: 180°C; MS(ES+): M⁺+H = 834.

### Beispiel 6:

### 2-Methyl-4-{4-[1-(3α,7α,12α-trihydroxy-10β,13β-dimethyl-17β-(pentansäure-4-yl)-hexadecahydro-cyclopenta[a]phenthren-3-yl)-ethin-2-yl]-benzyl}-amino-chinolin-3-carbonsäure-methylester

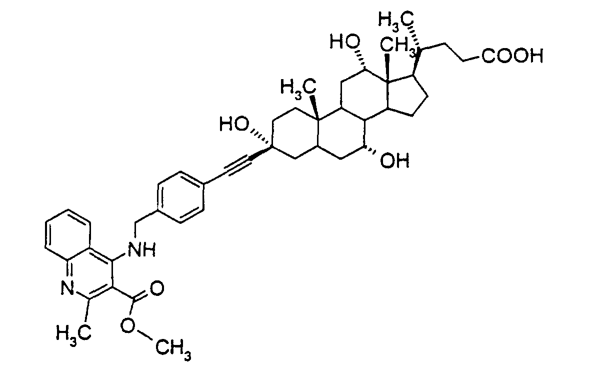

Darstellung der Zwischenprodukte:
Zwischenprodukt 1:3β-Acetylen-cholsäure 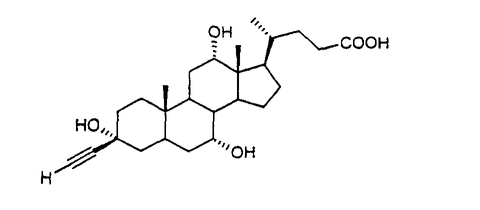 siehe Beispiel 1

### Zwischenprodukt 2: 4-(4-Bromo-benzylamino)-2-methyl-chinolin-3-carbonsäuremethylester

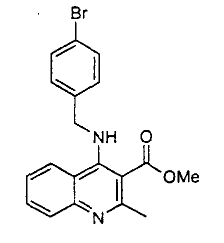

### Syntheseweg:

a) 4-Amino-2-methyl-chinolin-3-carbonsäure-methylester kann in dem Fachmann bekannter Weise hergestellt werden (Tetrahedron 51 (1995), 12277).
b) 4-(4-Bromo-benzylamino)-2-methyl-chinolin-3-carbonsäure-methylester wird nach dem gleichen Verfahren synthetisiert, wie bereits für 1-(4-(4-Bromobenzylamino-2-methyl-chinolin-3-yl)-ethanon (Beispiel 1, Zwischenprodukt 2c) beschrieben wurde, ausgehend von 4-Amino-2-methyl-chinolin-3-carbonsäuremethylester und 4-Bromo-benzylbromid. Schmp.: 89°C; MS(ES+): 385/387.

2-Methyl-4-{4-[1-(3α,7α,12α-trihydroxy-10β,13β-dimethyl-17β-(pentansäure-4-yl)-hexadecahydro-cyclopenta[a]phenthren-3-yl)-ethin-2-yl]-benzy}-amino-chinolin-3-carbonsäure-methylester wird nach dem angegebenen allgemeinen Verfahren (s. Beispiel 1) hergestellt. Schmp.: 165°C; MS(FAB): 737.

### Beispiel 7:

### 2-Methyl-4-{4-[1-(3α,7α,12α,trihydroxy-10β,13β-dimethyl-17β-(pentansäure-4-yl)-hexadecahydro-cyclopenta[a]phenanthren-3-yl)-ethin-2-yl]-benzyl)-amino-chinolin-3-carbonsäure-n-butylester

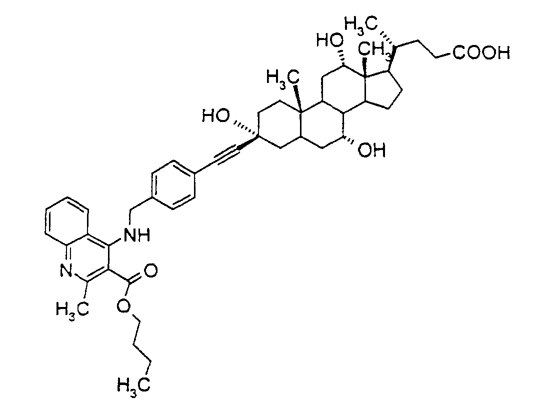

Darstellung der Zwischenprodukte:
Zwischenprodukt 1:3β-Acetylen-cholsäure 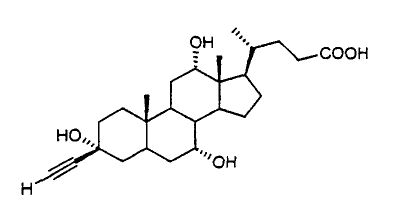 siehe Beispiel 1

### Zwischenprodukt 2: 4-(4-Bromo-benzylamino)-2-methyl-chinolin-3-carbonsäure-nbutylester

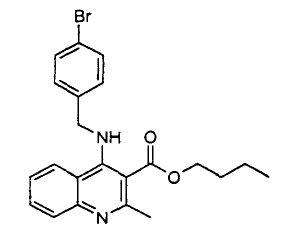

### Syntheseweg:

a) 4-(4-Bromo-benzylamino)-2-methyl-chinolin-3-carbonsäure-n-butylester 200 mg (1.0 eq) 4-(4-Bromo-benzylamino)-2-methyl-chinolin-3-carbonsäuremethylester (s. Beispiel 5) werden in einem n-Butanol / THF-Gemisch gelöst und bei Raumtemp. mit 2.5 eq NaH (55 %ig) versetzt und unter Feuchtigkeitsausschluß 2 h gerührt. Zur Aufarbeitung wird vom Lösungsmittel befreit und der Rückstand in CH₂Cl₂ aufgenommen. Es wird mit H₂O gewaschen und die wäßrige Phase noch einmal mit CH₂Cl₂ extrahiert. Die vereinigten organischen Phasen werden mit ges. NaHCO₃-Lösung gewaschen und mit MgSO₄ getrocknet. Chromatographie an Kieselgel (Essigester / n-Heptan 1:1 → 4:1) liefert den Produktester als farblosen Feststoff. Schmp.:
   116°C; MS(ES+): 427/429.

2-Methyl-4-{4-[1-(3α,7α,12α-trihydroxy-10β,13β-dimethyl-17β-(pentansäure-4-yl)-hexadecahydro-cyclopenta[a]phenthren-3-yl)-ethin-2-yl]-benzyl}-amino-chinolin-3-carbonsäure-n-butylester wird nach dem angegebenen allgemeinen Verfahren (s. Beispiel 1) hergestellt. Schmp.: 70°C; MS(FAB): 779.

### Beispiel 8:

### 2-Methyl-4-{4-[1-(3α,7α,12α-trihydroxy-10β,13β-dimethyl-17β-(pentansäure-4-yl)-hexadecahydro-cyclopenta[a]phenthren-3-yl)-ethin-2-yl]-benzyl}-amino-chinolin-3-carbonsäure-isopropylester

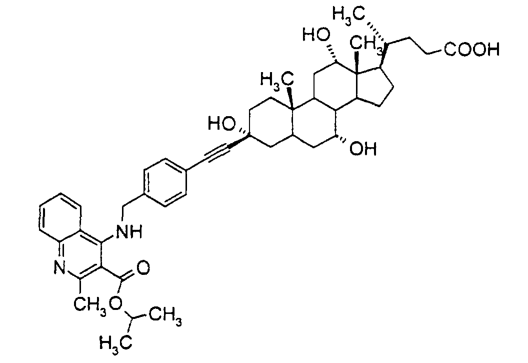

Darstellung der Zwischenprodukte:
Zwischenprodukt 1: 3β-Acetylen-cholsäure 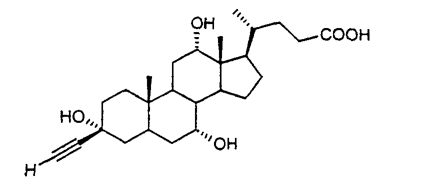 siehe Beispiel 1

### Zwischenprodukt 2: 4-(4-Bromo-benzylamino)-2-methyl-chinolin-3-carbonsäureisopropylester

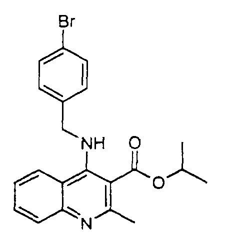

### Syntheseweg:

a) 4-(4-Bromo-benzylamino)-2-methyl-chinolin-3-carbonsäure-isopropylester wird analog zu 4-(4-Bromo-benzylamino)-2-methyl-chinolin-3-carbonsäure-nbutylester (s. Beispiel 6) durch Umesterung des entsprechenden Methylesters in einem Isopropanol / THF-Gemisch hergestellt, wobei die Titelverbindung als farbloses Öl erhalten wird. MS(ES+): 413/415.

2-Methyl-4-{4-[1-(3α,7α,12α-trihydroxy-10β,13β-dimethyl-17β-(pentansäure-4-yl)-hexadecahydro-cyclopenta[a]phenthren-3-yl)-ethin-2-yl]-benzyl}-amino-chinolin-3-carbonsäure-isopropylester wird nach dem angegebenen allgemeinen Verfahren (s. Beispiel 1) hergestellt. Schmp.: 150°C; MS(FAB): 765.

### Beispiel 9:

### 2-Methyl-4-benzylamino-6-{2-[3α,7α,12α-trihydroxy-10β,13β-dimethyl-17β-(pentansäure-4-yl)-hexadecahydro-cyclopenta[a]phenthren-3-yl]-ethin-1-yl}-chinolin-3-carbonsäure-methylester

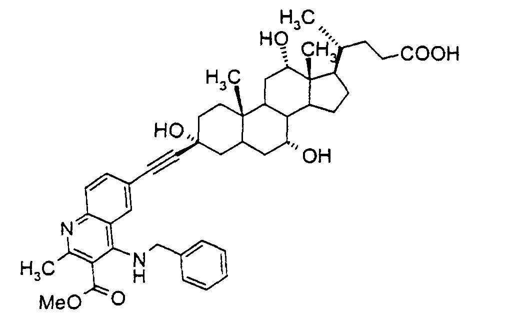

Darstellung der Zwischenprodukte:
Zwischenprodukt 1: 3β-Acetylen-cholsäure 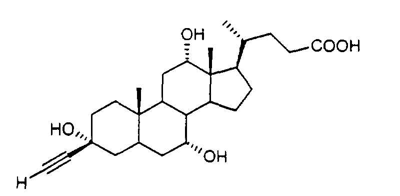 siehe Beispiel 1

### Zwischenprodukt 2: 4-(4-Bromo-benzylamino)-2-methyl-chinolin-3-carbonsäureisopropylester

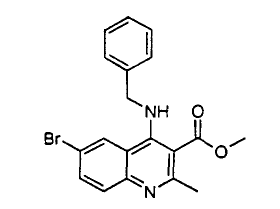

### Syntheseweg:

a) 5-Bromo-2-amino-benzonitril kann in literaturbekannter Weise hergestellt werden (Synlett, 1994, 450); MS(Cl): 197/199.
b) 6-Bromo-4-amino-2-methyl-chinolin-3-carbonsäure-methylester kann in dem Fachmann bekannter Weise hergestellt werden (Tetrahedron 51 (1995), 12277).
c) 6-Bromo-4-benzylamino-2-methyl-chinolin-3-carbonsäure-methylester wird nach dem gleichen Verfahren synthetisiert, wie bereits für 1-(4-(4-Bromo-benzylamino-2-methyl-chinolin-3-yl)-ethanon (Beispiel 1, Zwischenprodukt 2c) beschrieben wurde, ausgehend von 6-Bromo-4-amino-2-methyl-chinolin-3-carbonsäuremethylester und 4-Bromo-benzylbromid. Schmp.: 89°C; MS(ES+): 385/387.

2-Methyl-4-benzylamino-6-{2-[3α,7α,12α-trihydroxy-10β,13β-dimethyl-17β-(pentansäure-4-yl)-hexadecahydro-cyclopenta[a]phenthren-3-yl]-ethin-1-yl}-chinolin-3-carbonsäure-methylester wird nach dem angegebenen allgemeinen Verfahren (s. Beispiel 1) hergestellt. Schmp.: 223°C (Zers.); MS(ES+): 737.

### Beispiel 10:

### 2-Methyl-4-benzylamino-6-{2-[3α,7α,12α-trihydroxy-10β,13β-dimethyl-17β-(pentansäure-4-yl)-hexadecahydro-cyclopenta[a]phenthren-3-yl]-ethylen-1-yl}chinolin-3-carbonsäure-methylester

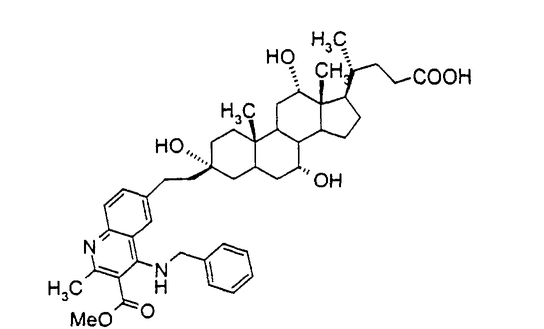

Das in Beispiel 8 beschriebene Produkt wird analog zu der in Beispiel 2 angegebenen Methode hydriert, wobei die Zielverbindung als leicht gelber Feststoff erhalten wird. Schmp.: > 185 °C (Zersetzung); MS(FAB): 741.

### Beispiel 11

### 2,5-Dimethyl-4-{4-[1-(3α,7α,12α-trihydroxy-10β,13β-dimethyl-17β-(pentansäure-4-yl)-hexadecahydro-cyclopenta[a]phenthren-3-yl)-ethin-2-yl]-benzyl}-amino-chinolin-3-carbonsäure-methylester

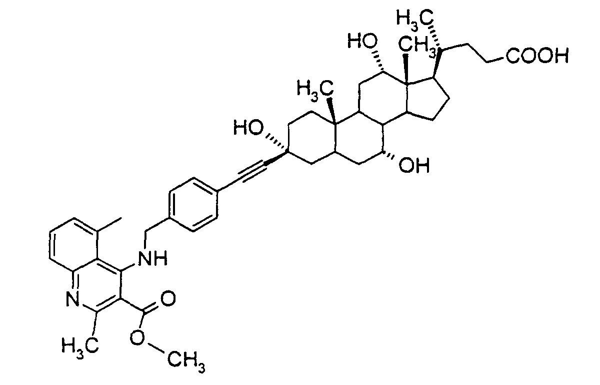

Darstellung der Zwischenprodukte:
Zwischenprodukt 1:3β-Acetylen-cholsäure
siehe Beispiel 1

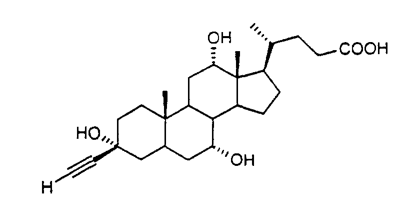

### Zwischenprodukt 2: 4-(4-Bromo-benzylamino)-2,5-dimethyl-chinolin-3-carbonsäuremethylester

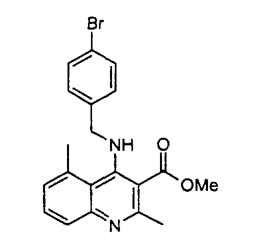

### Syntheseweg:

a) 4-Amino-2,5-dimethyl-chinolin-3-carbonsäure-methylester kann analog zu Zwischenprodukt 2a, Bsp. 6, in dem Fachmann bekannter Weise hergestellt werden (Tetrahedron 51 (1995), 12277).
b) 4-(4-Bromo-benzylamino)-2,5-dimethyl-chinolin-3-carbonsäure-methylester wird nach dem gleichen Verfahren synthetisiert, wie bereits für 1-(4-(4-Bromobenzylamino-2-methyl-chinolin-3-yl)-ethanon (Beispiel 1, Zwischenprodukt 2c) beschrieben wurde, ausgehend von 4-Amino-2,5-dimethyl-chinolin-3-carbonsäure-methylester und 4-Bromo-benzylbromid. Schmp.: 150°C; MS(Cl+): 399/401.

2,5-Dimethyl-4-{4-[1-(3α,7α,12α-trihydroxy-10β,13β-dimethyl-17β-(pentansäure-4-yl)-hexadecahydro-cyclopenta[a]phenanthren-3-yl)-ethin-2-yl]-benzyl}-aminochinolin-3-carbonsäure-methylester wird nach dem angegebenen allgemeinen Verfahren (s. Beispiel 1) hergestellt. Schmp.: >155°C; MS(ES+): M⁺+H = 751.

### Beispiel 12:

### 2,5-Dimethyl-4-{4-[1-(3α,7α,12α-trihydroxy-10β,13β-dimethyl-17β-(pentansäure-4-yl)-hexadecahydro-cyclopenta[a]phenanthren-3-yl)-ethylen-2-yl]-benzyl}-aminochinolin-3-carbonsäure-methylester

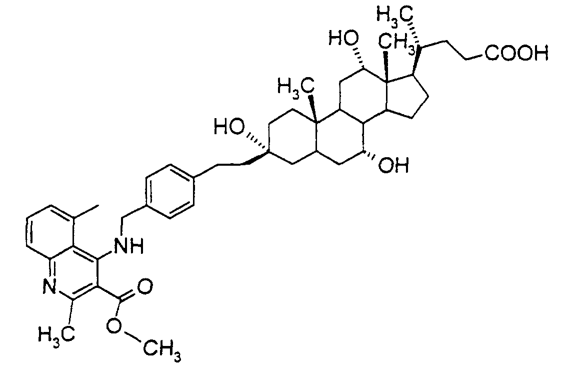

Das in Beispiel 11 beschriebene Produkt wird analog zu der in Beispiel 2 angegebenen Methode hydriert, wobei die Zielverbindung als leicht gelber Feststoff erhalten wird. Schmp.: > 180 °C Zersetzung; MS(ES+): M⁺+H = 756.

### Beispiel 13:

### 2-Methyl-3-(aceto-1-yl)-4-{3-[1-(3α,7α,12α-trihydroxy-10β,13β -dimethyl-17β-(pentansäure-4-yl)hexadecahydro-cyclopenta[a]phenanthren-3-yl)-ethin-2-yl]-benzyl}-amino-chinolin

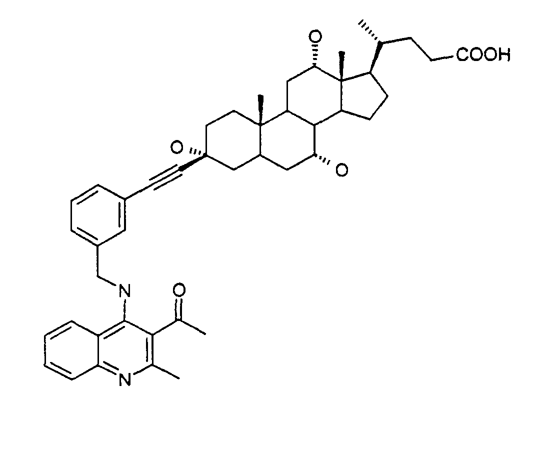

Darstellung der Zwischenprodukte:
Zwischenprodukt 1: 3β-Acetylen-cholsäure 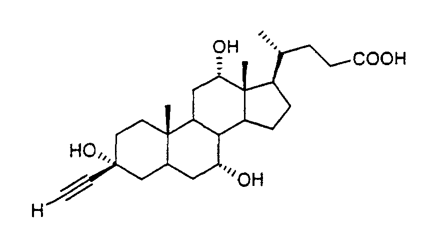 siehe Beispiel 1

### Zwischenprodukt 2: 1-(4-(3-Bromobenzyl)amino-2-methyl-chinolin3-yl)-ethanon

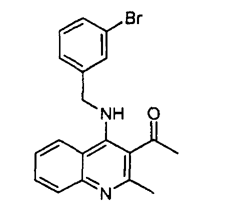

### Syntheseweg:

a) 1-(4-Amino-2-methyl-chinolin-3-yl)-ethanon kann analog zu Zwischenprodukt 2a, Bsp. 6, durch Zinntetrachlorid-vermittelte Umsetzung von 2-Aminobenzonitril und Acetylaceton in literaturbekannter Weise hergestellt werden (Tetrahedron 51 (1995), 12277).
b) 1-(4-(3-Bromobenzyl-)amino-2-methyl-chinolin3-yl)-ethanon wird nach dem gleichen Verfahren synthetisiert, wie bereits für 1-(4-(4-Bromobenzyl-)amino-2-methyl-chinolin-3-yl)-ethanon (Beispiel 1, Zwischenprodukt 2c) beschrieben wurde, ausgehend von 1-(4-Amino-2-methyl-chinolin3-yl)-ethanon und 3-Bromo-benzylbromid. Schmp.: 109°C; MS(ES+): 369/371.

2-Methyl-3-(aceto-1-yl)-4-{3-[1-(3α,7α,12α -trihydroxy-10β,13β -dimethyl-17β-(pentansäure-4-yl)-hexadecahydro-cyclopenta[a]phenanthren-3-yl)-ethin-2-yl]-benzyl}-amino-chinolin wird nach dem angegebenen allgemeinen Verfahren (s. Beispiel 1) hergestellt. Schmp.: >195°C Zersetzung; MS(ES+): M⁺+H = 721.

### Beispiel 14:

### 2-Met hyl-3-(aceto-1-yl)-4-{3-[1-(3α,7α,12α-trihydroxy-10β,13β-dimethyl-17β-(pentansäure-4-yl)-hexadecahydro-cyclopenta[a]phenanthren-3-yl)-ethylen-2-yl]-benzyl}-amino-chinolin

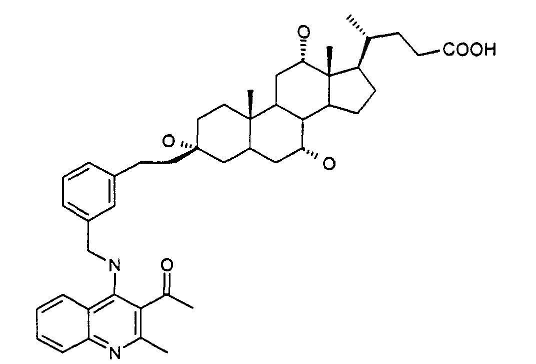

Die Titelverbindung wird durch katalytische Hydrierung des in Beispiel 13 beschriebenen Produktes, wie in Beislpiel 2 beschrieben, erhalten. Schmp.: >170°C Zersetzung; MS(FAB+): M⁺ = 725.

### Beispiel 15:

### 2-Methyl-3-(aceto-1-yl)-4-{3-[1-(3α,7α,12α-trihydroxy-10β,13β-dimethyl-17β-(pentansäure-N-glycylamid-4-yl)-hexadecahydro-cyclopenta[a]phenanthren-3-yl)-ethylen-2-yl]-benzyl}-amino-chinolin

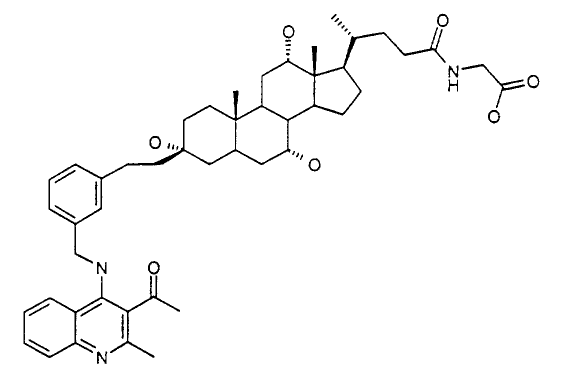

### Syntheseweg:

a) 2-Methyl-3-(aceto-1-yl)-4-{3-[1-(3α,7α,12α-trihydroxy-10β,13β-dimethyl-17β-(pentansäure-(N-glycidylethylester)-amid-4-yl)-hexadecahydrocyclopenta[a]phenanthren-3-yl)-ethylen-2-yl]-benzyl}-amino-chinolin.
   55 mg des in Beispiel 14 hergestellten Produktes werden in 5 ml abs. DMF vorgelegt und bei 0 °C mit 1 ml einer Lösung von 0.1 ml Triethylamin in 10 ml abs. DMF versetzt. Nach Zugabe von 25 mg TOTU wird 20 min bei 0 °C, sowie 30 min bei Raumtemperatur gerührt. Diese Lösung wird zu einer Lösung bestehend aus 11 mg Glycinethylester-hydrochlorid in 2 ml DMF, 2 ml H₂O und 1 ml Triehtylamin gegeben und bei Raumtemperatur gerührt, bis vollständiger Umsatz erreicht ist. Das Lösungsmittel wird abdestilliert und der Rückstand an Kieselgel chromatographiert, wobei 41 mg des entsprechenden Ethylesters erhalten werden.
b) 2-Methyl-3-(aceto-1-yl)-4-{3-[1-(3α,7α,12α-trihydroxy-10β,13β -dimethyl-17β-(pentansäure-N-glycylamid-4-yl)-hexadecahydro-cyclopenta[a]phenanthren-3-yl)-ethylen-2-yl]-benzyl}-amino-chinolin.
   41 mg des hergestellten Ethylesters werden in 5 ml Methanol gelöst und bei Raumtemperatur eine Lösung von 28 mg KOH in 1.5 ml H₂O zugetropft und bei Raumtemperatur gerührt. Nach 2 Stunden wird vom Lösungsmittel befreit, der Rückstand in 5 ml H₂O aufgenommen und mit verd. HCl ein pH-Wert von 5 eingestellt, worauf das gewünschte Produkt ausfällt. Der Niederschlag wird abgesaugt und an der Luft getrocknet. MS(ES+): M⁺+H: 782.

### Beispiel 16:

### 2-Methyl-3-(aceto-1-yl)-4-{3-[1-(3α,7α,12α-trihydroxy-10β,13β-dimethyl-17β-(pentansäure-N-(2-sulfonsäure)-ethylamid-4-yl)-hexadecahydrocyclopenta[a]phenanthren-3-yl)-ethylen-2-yl]-benzyl}-amino-chinolin

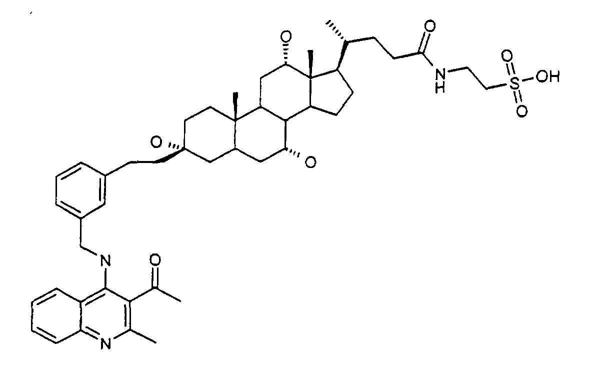

55 mg des in Beispiel 14 hergestellten Produktes werden in 5 ml abs. DMF vorgele und bei 0 °C mit 1 ml einer Lösung von 0.1 ml Triethylamin in 10 ml abs. DMF versetzt. Nach Zugabe von 25 mg TOTU wird 20 min bei 0 °C, sowie 30 min bei Raumtemperatur gerührt. Diese Lösung wird zu einer Lösung bestehend aus 10 mg Taurin in 2 ml DMF, 2 ml H₂O und 1 ml Triehtylamin gegeben und bei Raumtemperatur gerührt, bis vollständiger Umsatz erreicht ist. Es wird vom Lösungsmittel befreit und der Rückstand an Kieselgel chromatographiert, wobei 36 mg der Titelverbindung in Form eines leicht gelblichen Feststoffs erhalten werden.
MS(ES+): M⁺+H: 832.

### Beispiel 17:

### 2-Methyl-3-(aceto-1-yl)-4-{2-[1-(3α,7α,12α-trihydroxy-10β,13β -dimethyl-17β-(pentansäure-4-yl)-hexadecahydro-cyclopenta[a]phenanthren-3-yl)-ethin-2-yl]-benzyl}-amino-chinolin

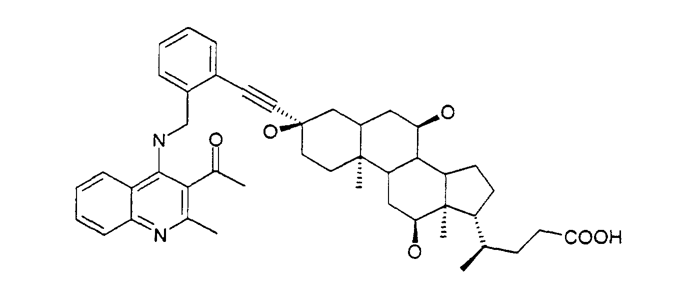

Darstellung der Zwischenprodukte:
Zwischenprodukt 1: 3β-Acetylen-cholsäure 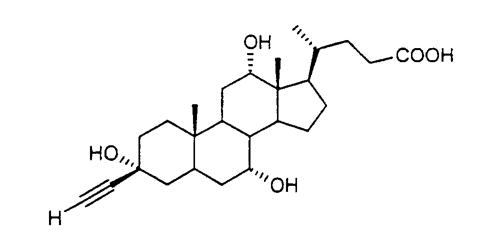 siehe Beispiel 1

### Zwischenprodukt 2:1-(4-(3-Bromobenzyl)amino-2-methyl-chinolin3-yl)-ethanon

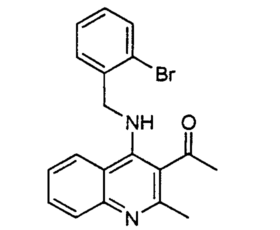

### Syntheseweg:

a) 4-Amino-2-methyl-chinolin-3-yl)-ethanon: s. Beispiel 13, (Tetrahedron 51 (1995), 12277).
b) 4-(2-Bromobenzyl-)amino-2-methyl-chinolin3-yl)-ethanon wird nach dem gleichen Verfahren synthetisiert, wie bereits für 1-(4-(4-Bromobenzyl-)amino-2-methylchinolin-3-yl)-ethanon (Beispiel 1, Zwischenprodukt 2c) beschrieben wurde, ausgehend von 1-(4-Amino-2-methyl-chinolin3-yl)-ethanon und 2-Bromo-benzylbromid. Schmp.: 134°C; MS(ES+): 369/371.

2-Methyl-3-(aceto-1-yl)-4-{2-[1-(3α,7α,12α-trihydroxy-10β,13β-dimethyl-17β-(pentansäure-4-yl)-hexadecahydro-cyclopenta[a]phenanthren-3-yl)-ethin-2-yl]-benzyl}-amino-chinolin wird nach dem angegebenen allgemeinen Verfahren (s. Beispiel 1) aus den beiden Zwischenprodukten hergestellt. MS(ES+): M⁺+H = 721.

### Beispiel 18:

### 2-Methyl-3-(aceto-1-yl)-4-{2-[1-(3α,7α,12α-trihydroxy-10β,13β-dimethyl-17β-(pentansäure-4-yl)-hexadecahydro-cyclopenta[a]phenanthren-3-yl)-ethylen-2-yl]-benzyl}-amino-chinolin

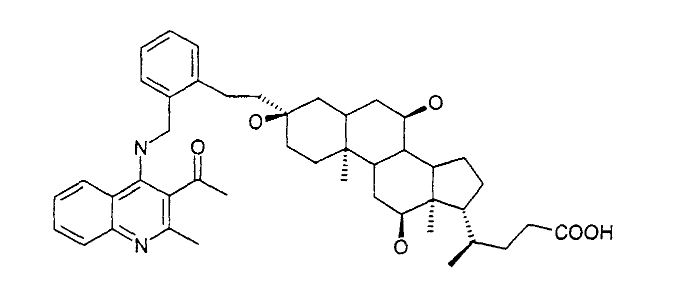

Hydrierung der Produktverbindung aus Beispiel 17 nach der in Beispiel 2 aufgeführten Methode lierfert das gewünschte Produkt in Form eines leicht gelblichen Festsoffs. Schmp.: > 165 °C Zersetzung; MS(ES+): 725.

### Beispiel 19:

### 4-[3β-(2-{4-[(3-Acetyl-2-methyl-chinolin-4-ylamino)-methyl]-benzoylamino)-ethoxy)-7α,12α-dihydroxy-10β,13β-dimethyl-hexadecahydrocyclopenta[a]phenanthren-17β-yl]-pentansäuremethylester.

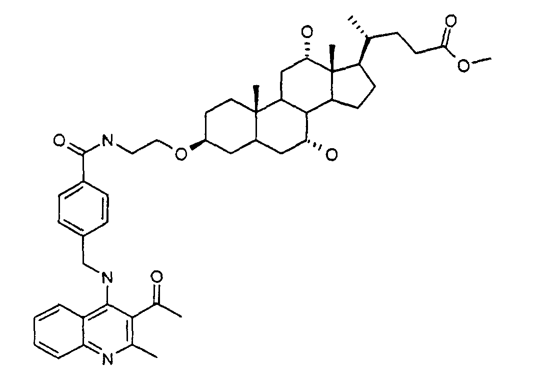

Darstellung der Zwischenprodukte:

### Zwischenprodukt 1: 3β-O-(1-amino-et-2-yl)-cholsäuremethylester

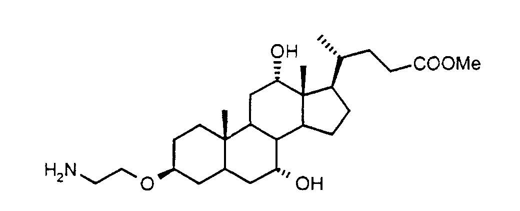

s. Beispiel 3. (Tetrahedron Lett., 33 (1992), 195; Tetrahedron Lett., 34 (1993), 817).

### Zwischenprodukt 2: 4-[(3-Acetyl-2-methyl-chinolin-4-ylamino)-methyl]-benzoesäure

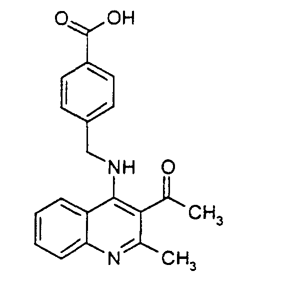

### Syntheseweg:

a) 2-Methyl-3-(aceto-1-yl)-4-(4-bromobenzyl)-amino-chinolin ist in Beispiel 1 als Zwischenprodukt 2 beschrieben.
b) 4-[(3-Acetyl-2-methyl-chinolin-4-ylamino)-methyl]-benzoesäure.

370 mg 2-Methyl-3-(aceto-1-yl)-4-(4-bromobenzyl)-amino-chinolin, 79 mg Triphenylphosphin und 78 mg Calciumformiat werden in 4 ml DMF und 4 ml Benzol gelöst. Unter Schutzgas werden 9 mg Palladiumacetat zugegeben und die Lösung unter CO-atmosphäre auf 120 °C erhitzt. Nach zwei Stunden werden 58 mg Tetrakistriphenylphosphin-palladium zugegeben und weiter bei 120 °C gerührt. Es wird solange bei der angegebenen Temperatur weitergerührt, bis keine Umsatzsteigerung mehr feststellbar ist. Evtl. wird zusätzlicher Palladium-Katalysator zugegeben. Zur Aufarbeitung wird mit 2 N NaOH versetzt und mit Dichlormethan extrahiert. Die organische Phase wird noch einmal mit 2 N NaOH extrahiert und die vereinigten wässrigen Phasen mit Dichlormethan extrahiert. Die NaOH-Extrakte werden mit 6 N HCl auf einen pH-Wert von 6 eingestellt und i. Vak. eingeengt. Der Rückstand wird in wenig Methanol aufgenommen. Es wird von unlöslichen Salzen abfiltriert. Das Filtrat wird eingeengt und in wenig H₂O aufgenommen. Das Produkt fällt dabei als gelber unlöslicher Feststoff an, dar abgsaugt und an der Luft getrocknet wird. Man erhält 81 mg eines gelben Feststoffs. Schmp.: > 210 °C
Zersetzung; MS(ES+): 335.

4-[3β-(2-{4-[(3-Acetyl-2-methyl-chinolin-4-ylamino)-methyl]-benzoylamino)-ethoxy)-7α,12α-dihydroxy-10β,13β-dimethyl-hexadecahydrocyclopenta[a]phenanthren-17β-yl]-pentansäuremethylester.
67 mg des Zwischenprodukts 2 werden in 5 ml DMF gelöst und bei 0 °C mit 1 ml einer Lösung von 0.28 ml Triethylamin in 10 ml DMF versetzt. Nach Zugabe einer Lösung von 66 mg TOTU in 2 ml DMF wird 30 min bei 0 °C und weitere 45 min bei Raumtemp. gerührt. Diese Lösung wird zu einer zweiten Lösung von 93 mg des Zwischenprodukts 1 in 2 ml DMF und 1 ml Triethylamin getropft und bei Raumtemp. gerührt, bis vollständiger Umsatz festgestellt wird. Zur Aufarbeitung wird i. Vak. eingeengt und der Rückstand in Essigester augenommen. Der Niederschlag wird abfiltriert und das Filtrat mit NaHCO₃-Lösung und mit H₂O gewaschen. Die organische Phase wird abgetrennt, mit MgSO₄ getrocknet und vom Lösungsmittel befreit. Der so erhaltene Rückstand wird zusammen mit dem abgetrennten Niederschlag an Kieselgel chromatographiert, wobei 71 mg des Produkt als gelboranger Feststoff erhalten werden. Schmp.: > 98 °C Zersetzung; MS(ES+): 782.

### Beispiel 20:

### 4-[3β-(2-{4-[(3-Acetyl-2-methyl-chinolin-4-ylamino)-methyl]-benzoylamino)-ethoxy)-7α,12α-dihydroxy-10β,13β-dimethyl-hexadecahydrocyclopenta[a]phenanthren-17β-yl]-pentansäure.

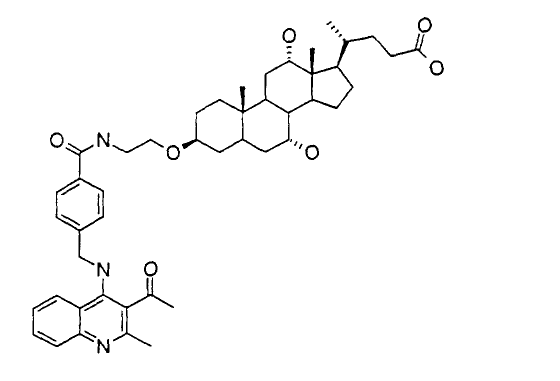

Der in Beispiel 19 erhaltene Ester wird analog zu der in Beispiel 4 beschriebenen Verseifung umgesetzt, wobei das gewünschte Produkt als farbloser Feststoff erhalten wird. Schmp.: > 145 °C Zersetzung; MS(ES+): 768.

### Beispiel 21

### 2-Ethyl-4-{4-[1-(3α,7α,12α-trihydroxy-10β,13β-dimethyl-17β-(pentansäure-4-yl)-hexadecahydro-cyclopenta[a]phenanthren-3-yl)-ethin-2-yl)-benzyl}-amino-chinolin-3-carbonsäure-methylester

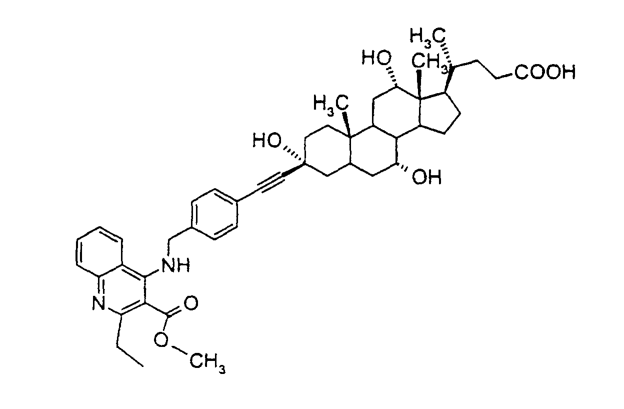

Darstellung der Zwischenprodukte:
Zwischenprodukt 1:3β-Acetylen-cholsäure 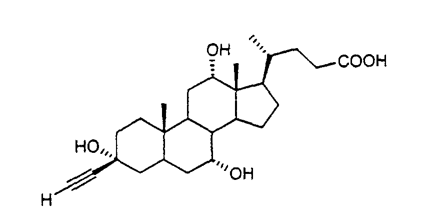 siehe Beispiel 1

### Zwischenprodukt 2: 4-(4-Bromo-benzylamino)-2-ethyl-chinolin-3-carbonsäuremethylester

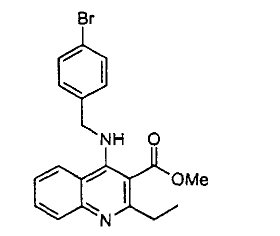

### Syntheseweg:

a) 4-Amino-2-ethyl-chinolin-3-carbonsäure-methylester kann in dem Fachmann bekannter Weise hergestellt werden (Tetrahedron 51 (1995), 12277).
b) 4-(4-Bromo-benzylamino)-2-ethyl-chinolin-3-carbonsäure-methylester wird nach dem gleichen Verfahren synthetisiert, wie bereits für 1-(4-(4-Bromo-benzylamino-2-methyl-chinolin-3-yl)-ethanon (Beispiel 1, Zwischenprodukt 2c) beschrieben wurde, ausgehend von 4-Amino-2-ethyl-chinolin-3-carbonsäure-methylester und 4-Bromo-benzylbromid. MS(ES+): 399/401.

2-Ethyl-4-{4-(1-(3α,7α,12α-trihydroxy-10β,13β-dimethyl-17β-(pentansäure-4-yl)-hexadecahydro-cyclopenta[a]phenanthren-3-yl)-ethin-2-yl]-benzyl}-amino-chinolin-3-carbonsäure-methylester wird nach dem angegebenen allgemeinen Verfahren (s. Beispiel 1) hergestellt. Schmp.: >200 °C; MS(FAB): 751.

### Beispiel 22:

### 2-Ethyl-4-{4-[1-(3α,7α,12α-trihydroxy-10β,13β-dimethyl-17β-(pentansäure-4-yl)-hexadecahydro-cyclopenta[a]phenanthren-3-yl)-ethylen-2-yl]-benzyl}-amino-chinolin-3-carbonsäure-methylester

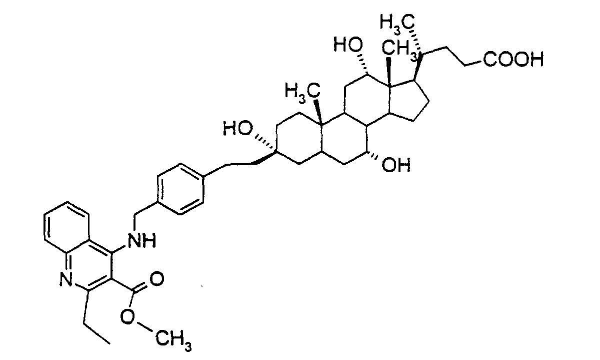

Das in Beispiel 21 beschriebene Produkt wird analog zu der in Beispiel 2 angegebenen Methode hydriert, wobei die Zielverbindung als leicht gelber Feststoff erhalten wird. Schmp.: > 190 °C (Zersetzung); MS(ES+): 755.

### Beispiel 23:

### 2-Methyl-3-(aceto-1-yl)-4-{4-[1-(3α,7α,12α-trihydroxy-10β,13β -dimethyl-17β-(pentansäure-4-yl)-hexadecahydro-cyclopenta[a]phenanthren-3-yl)-ethin-2-yl]-3-fluoro-benzyl}-amino-chinolin

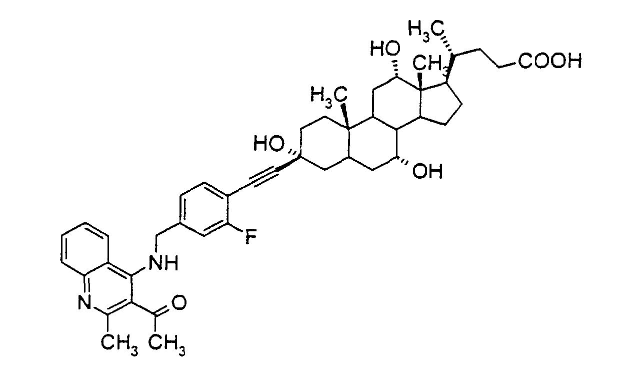

Darstellung der Zwischenprodukte:
Zwischenprodukt 1:3β-Acetylen-cholsäure 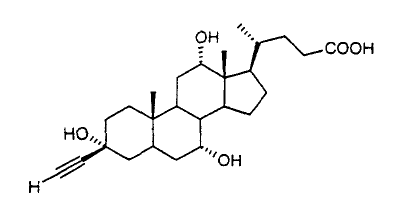 siehe Beispiel 1

### Zwischenprodukt 2:1-(4-(4-Bromo-3-fluoro-benzyl)amino-2-methyl-chinolin3-yl)-ethanon

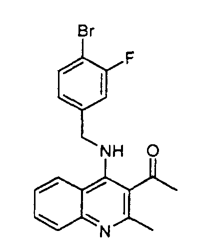

### Syntheseweg:

a) 1-(4-Amino-2-methyl-chinolin-3-yl)-ethanon; s. Beispiel 13 (Tetrahedron 51 (1995), 12277).
b) 1-(4-(4-Bromo-3-fluoro-benzyl-)amino-2-methyl-chinolin3-yl)-ethanon wird nach dem gleichen Verfahren synthetisiert, wie bereits für 1-(4-(4-Bromobenzyl)-amino-2-methyl-chinolin-3-yl)-ethanon (Beispiel 1, Zwischenprodukt 2c) beschrieben wurde, ausgehend von 1-(4-Amino-2-methyl-chinolin-3-yl)-ethanon und 4-Bromo-3-fluoro-benzylbromid. MS(ES+): 387/389.

2-Methyl-3-(aceto-1-yl)-4-{4-[1-(3α,7α,12α-trihydroxy-10β,13β-dimethyl-17β-(pentansäure-4-yl)-hexadecahydro-cyclopenta[a]phenanthren-3-yl)-ethin-2-yl]-3-fluoro-benzyl}-amino-chinolin wird nach dem angegebenen allgemeinen Verfahren (s. Beispiel 1) hergestellt. MS(FAB): 739.

### Beispiel 24:

### 2-Methyl-3-(aceto-1-yl)-4-{4-[1-(3α,7α,12α-trihydroxy-10β,13β-dimethyl-17β-(pentansäure-4-yl)-hexadecahydro-cyclopenta[a]phenanthren-3-yl)-ethylen-2-yl]-3-fluoro-benzy)}-amino-chinolin

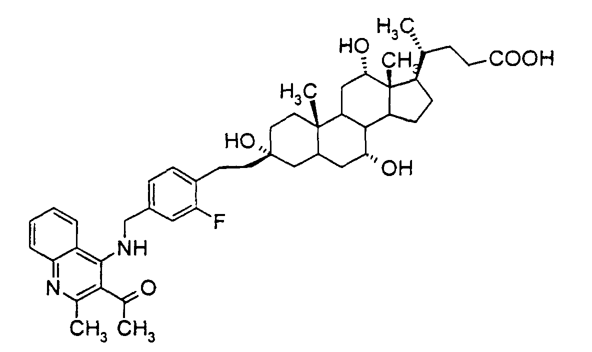

Das in Beispiel 23 beschriebene Produkt wird analog zu der in Beispiel 2 angegebenen Methode hydriert, wobei die Zielverbindung als leicht gelber Feststoff erhalten wird. MS(ES+): 744.

### Beispiel 25:

### 2-Methyl-3-(aceto-1-yl)-4-{4-[1-(3α,7α,12α-trihydroxy-10β,13β-dimethyl-17β-(pentansäure-4-yl)-hexadecahydro-cyclopenta[a]phenanthren-3-yl)-ethin-2-yl]-3-chloro-benzyl}-amino-chinolin

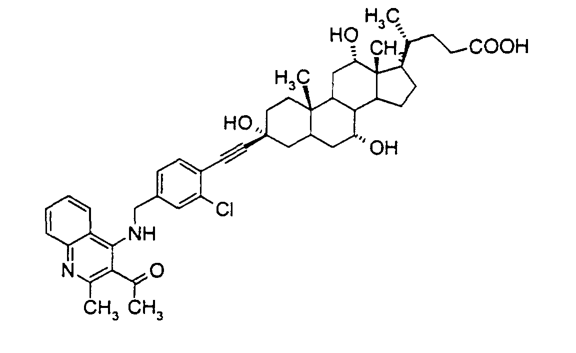

Darstellung der Zwischenprodukte:
Zwischenprodukt 1:3β-Acetylen-cholsäure 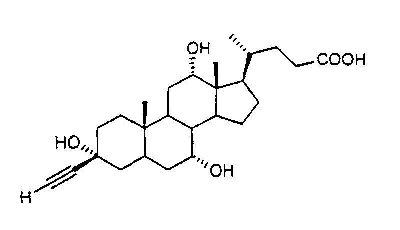 siehe Beispiel 1

### Zwischenprodukt 2:1-(4-(4-Bromo-3-chloro-benzyl)amino-2-methyl-chinolin-3-yl)-ethanon

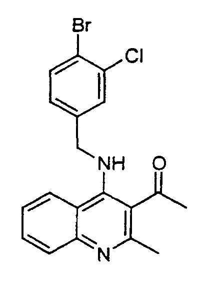

### Syntheseweg:

a) 1-(4-Amino-2-methyl-chinolin-3-yl)-ethanon; s. Beispiel 13 (Tetrahedron 51 (1995), 12277).
b) 1-(4-(4-Bromo-3-chloro-benzyl-)amino-2-methyl-chinolin-3-yl)-ethanon wird nach dem gleichen Verfahren synthetisiert, wie bereits für 1-(4-(4-Bromobenzyl)-amino-2-methyl-chinolin-3-yl)-ethanon (Beispiel 1, Zwischenprodukt 2c) beschrieben wurde, ausgehend von 1-(4-Amino-2-methyl-chinolin-3-yl)-ethanon und 4-Bromo-3-chloro-benzylbromid. MS(ES+):403/405.

2-Methyl-3-(aceto-1-yl)-4-{4-[1-(3α,7α,12α-trihydroxy-10β,13β -dimethyl-17β-(pentansäure-4-yl)-hexadecahydro-cyclopenta[a]phenanthren-3-yl)-ethin-2-yl]-3-chloro-benzyl}-amino-chinolin wird nach dem angegebenen allgemeinen Verfahren (s. Beispiel 1) hergestellt. MS(FAB): 755.

### Beispiel 26:

### 2-Methyl-3-(aceto-1-yl)-4-{4-[1-(3α,7α,12α -trihydroxy-10β,13β-dimethyl-17β-(pentansäure-4-yl)-hexadecahydro-cyclopenta[a]phenanthren-3-yl)-ethin-2-yl]-2-fluoro-benzyl}-amino-chinolin

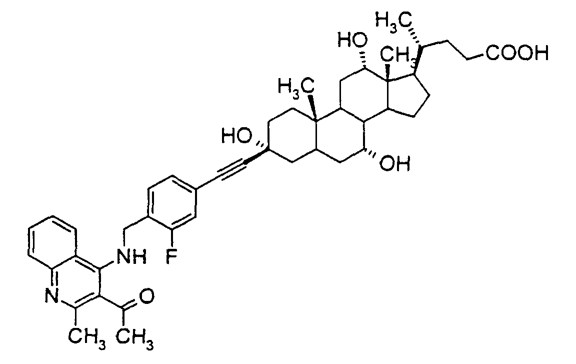

Darstellung der Zwischenprodukte:
Zwischenprodukt 1:3β-Acetylen-cholsäure 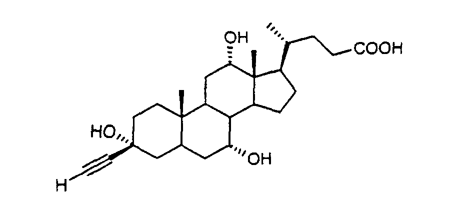 siehe Beispiel 1

### Zwischenprodukt 2:-(4-(4-Bromo-2-fluoro-benzyl)amino-2-methyl-chinolin-3-yl)-ethanon

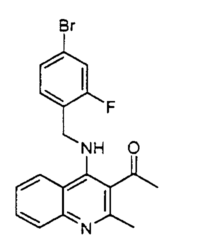

### Syntheseweg:

a) 1-(4-Amino-2-methyl-chinolin-3-yl)-ethanon; s. Beispiel 13 (Tetrahedron 51 (1995), 12277).
b) 1-(4-(4-Bromo-2-fluoro-benzyl-)amino-2-methyl-chinolin-3-yl)-ethanon wird nach dem gleichen Verfahren synthetisiert, wie bereits für 1-(4-(4-Bromobenzyl)-amino-2-methyl-chinolin-3-yl)-ethanon (Beispiel 1, Zwischenprodukt 2c) beschrieben wurde, ausgehend von 1-(4-Amino-2-methyl-chinolin-3-yl)-ethanon und 4-Bromo-2-fluoro-benzylbromid. MS(ES+): 387/389.

2-Methyl-3-(aceto-1-yl)-4-{4-(1-(3α,7α,12α-trihydroxy-10β,13β-dimethyl-17β-(pentansäure-4-yl)-hexadecahydro-cyclopenta[a]phenanthren-3-yl)-ethin-2-yl]-2-fluoro-benzyl}-amino-chinolin wird nach dem angegebenen allgemeinen Verfahren (s. Beispiel 1) hergestellt. MS(ES+): 739.

### Beispiel 27:

### 2-Methyl-3-(aceto-1-yl)-4-{4-(1-(3α,7α,12α-trihydroxy-10β,13β -dimethyl-17β-(pentansäure-4-yl)-hexadecahydro-cyclopenta[a]phenanthren-3-yl)-ethylen-2-yl]-2-fluoro-benzyl}-amino-chinolin

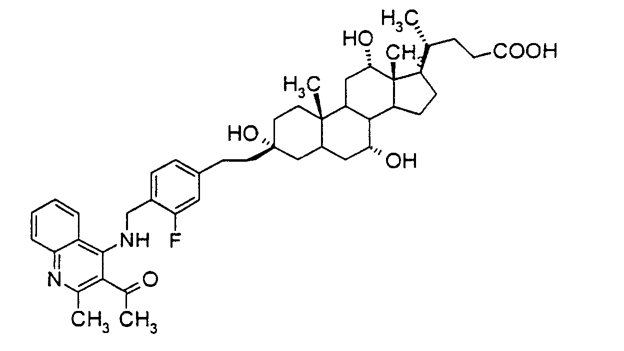

Das in Beispiel 26 beschriebene Produkt wird analog zu der in Beispiel 2 angegebenen Methode hydriert, wobei die Zielverbindung als leicht gelber Feststoff erhalten wird. MS(ES+): 744.

### Beispiel 28

### 2-Methyl-3-(aceto-1-yl)-4-{4-[1-(3α,7α,12α-trihydroxy-10β,13β -dimethyl-17β-(pentansäure-4-yl)-hexadecahydro-cyclopenta[a]phenanthren-3-yl)-ethin-2-yl]-2-chloro-benzyl}-amino-chinolin

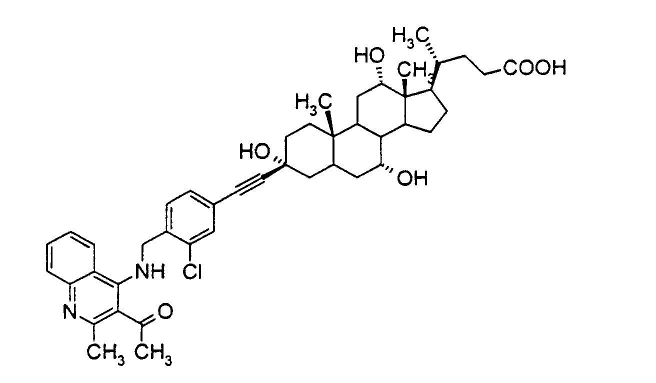

Darstellung der Zwischenprodukte:
Zwischenprodukt 1:3β-Acetylen-cholsäure 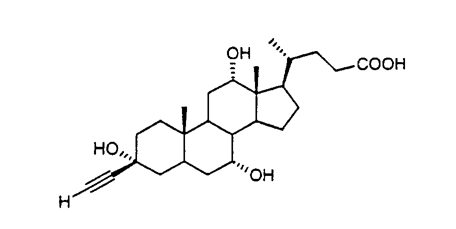 siehe Beispiel 1

### Zwischenprodukt 2:1-(4-(4-Bromo-2-chloro-benzyl)amino-2-methyl-chinolin-3-yl)-ethanon

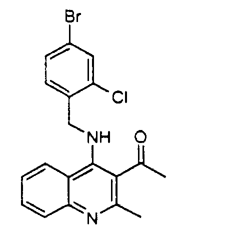

### Syntheseweg:

c) 1-(4-Amino-2-methyl-chinolin-3-yl)-ethanon; s. Beispiel 13 (Tetrahedron 51 (1995), 12277).
d) 1-(4-(4-Bromo-2-chloro-benzyl-)amino-2-methyl-chinolin-3-yl)-ethanon wird nach dem gleichen Verfahren synthetisiert, wie bereits für 1-(4-(4-Bromobenzyl)-amino-2-methyl-chinolin-3-yl)-ethanon (Beispiel 1, Zwischenprodukt 2c) beschrieben wurde, ausgehend von 1-(4-Amino-2-methyl-chinolin-3-yl)-ethanon und 4-Bromo-2-chloro-benzylbromid. MS(FAB+): 403/405.

2-Methyl-3-(aceto-1-yl)-4-{4-[1-(3α,7α,12α-trihydroxy-10β,13β-dimethyl-17β-(pentansäure-4-yl)-hexadecahydro-cyclopenta[a]phenanthren-3-yl)-ethin-2-yl]-2-chloro-benzyl}-amino-chinolin wird nach dem angegebenen allgemeinen Verfahren (s. Beispiel 1) hergestellt. MS(FAB+): 756.

### Beispiel 29:

### 2-Methyl-3-(aceto-1-yl)-4-{4-[1-(3α,7α,12+α-trihydroxy-10β,13β-dimethyl-17β-(pentansäure-4-yl)-hexadecahydro-cyclopenta[a]phenanthren-3-yl)-ethylen-2-yl]-2-chloro-benzyl}-amino-chinolin

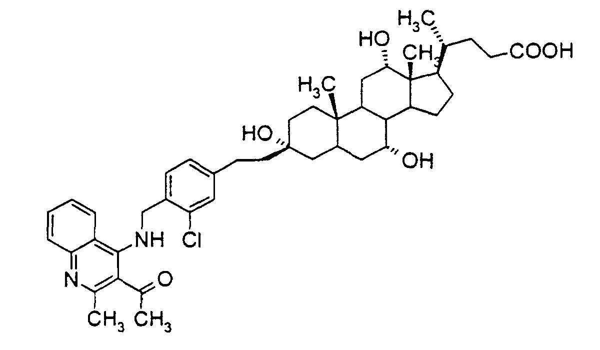

Das in Beispiel 28 beschriebene Produkt wird analog zu der in Beispiel 2 angegebenen Methode hydriert, wobei die Zielverbindung als leicht gelber Feststoff erhalten wird. MS(ES+): 760.

### Beispiel 30:

### 2,6-Dimethyl-3-acet-1-yl-4-{4-[1-(3α,7α,12α-trihydroxy-10β,13β-dimethyl-17β-(pentansäure-4-yl)-hexadecahydro-cyclopenta[a]phenanthren-3-yl-)ethin-2-yl]-benzyl}-amino-pyridin.

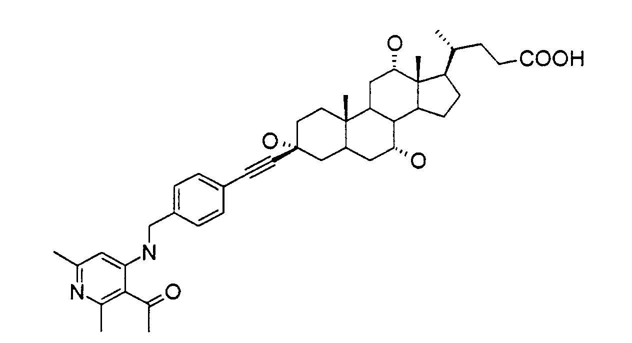

Darstellung der Zwischenprodukte:
Zwischenprodukt 1: 3β-Acetylen-cholsäure 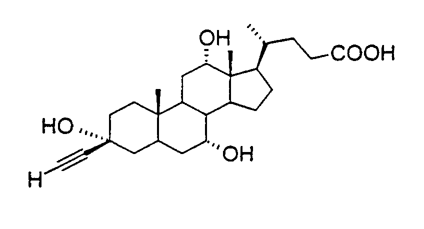 siehe Beispiel 1.

### Zwischenprodukt 2: 1-[4-(4-Bromo-benzylamino)-2,6-dimethyl-pyridin-3-yl]-ethanon

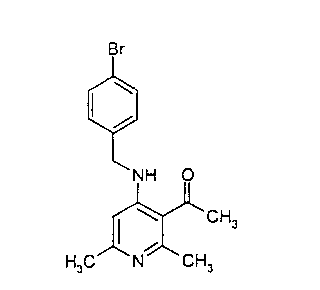

### Syntheseweg:

a) 3-Acetyl-2,6-dimethyl-1H-pyridin-4-on kann nach literaturbekanntem Verfahren hergestellt werden (Chem. Pharm. Bull., 31, 1983, 4303).
b) 1-(4-Chloro-2,6-dimethyl-pyridin-3-yl)-ethanon wird in dem Fachmann bekannter Weise synthetisiert (J. Heterocyclic Chem., 18, 1981, 603).
c) 1-[4-(4-Bromo-benzylamino)-2,6-dimethyl-pyridin-3-yl]-ethanon 1.0 g 1-(4-Chloro-2,6-dimethyl-pyridin-3-yl)-ethanone wird in 10 ml Dimethylacetamid gelöst und mit 1.5 Äquivalenten 4-Brom-benzylamin versetzt. Die Lösung wird auf 140 bis 150 °C erhitzt bis keine Umsatzsteigerung mehr feststellbar ist. Evtl. kann ein weiteres Äquivalent 4-Brom-benzylamin zugegeben werden. Zur Aufarbeitung wird das Lösungsmittel i. Vak. abdestilliert und der Rückstand an Kieselgel chromatographiert, wobei die Titelverbindung als gelbes Öl erhalten wird. MS(ES+): 333/335.

2,6-Dimethyl-3-acet-1-yl-4-{4-[1-(3α,7α,12α-trihydroxy-10β,13β-dimethyl-17β-(pentansäure-4-yl)-hexadecahydro-cyclopenta[a]phenanthren-3-yl-)ethin-2-yl]-benzyl}-amino-pyridin wird nach dem allgemeinen Verfahren (s. Beispiel 1) hergestellt. MS(ES+): 685.

### Beispiel 31:

### 2,6-Dimethyl-3-acet-1-yl-4-{4-[1-(3α,7α,12α -trihydroxy-10β,13β-dimethyl-17β-(pentansäure-4-yl)-hexadecahydro-cyclopenta[a]phenanthren-3-yl-)ethylen-2-yl]-benzyl}-amino-pyridin.

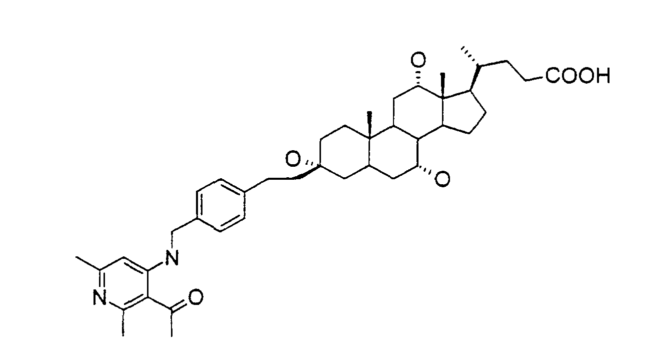

Das in Beispiel 30 erhaltene Produkt wird nach dem in Beispiel 2 beschriebenen Verfahren hydriert, wobei die Titelverbindung als leicht gelblicher Feststoff erhalten wird. MS(ES+): 689.

### Beispiel 32:

### 2-Ethyl-6-methyl-5-acet-1-yl-4-{4-[1-(3α,7α,12α-trihydroxy-10β,13β-dimethyl-17β-(pentansäure-4-yl)-hexadecahydro-cyclopenta[a]phenanthren-3-yl-)ethin-2-yl]-benzyl}-amino-pyridin.

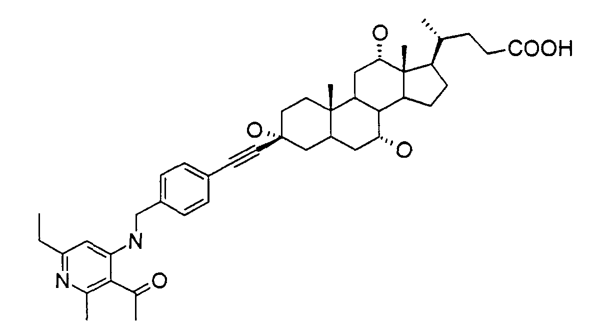

Darstellung der Zwischenprodukte:
Zwischenprodukt 1: 3β-Acetylen-cholsäure 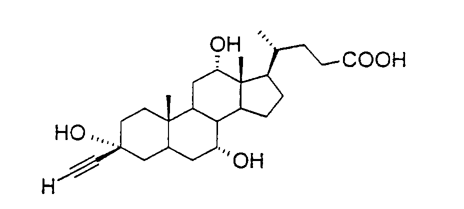 siehe Beispiel 1.

### Zwischenprodukt 2:1-[4-(4-Bromo-benzylamino)-2-ethyl-6-methyl-pyridin-5-yl]-ethanon

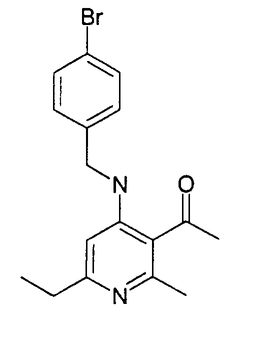

### Syntheseweg:

a) 2,2-Dimethyl-5-propionyl-[1,3]dioxane-4,6-dion wird in literaturbekannter Weise synthetisiert (J. Org. Chem., 43, 1978, 2087).
b) 3-Acetyl-6-ethyl-2-methyl-1H-pyridin-4-on wird in Anlehnung an die in Beispiel 30 beschriebene Methode (Beispiel 30; Zwischenprodukt 2a) synthetisiert (Chem. Pharm. Bull., 31, 1983, 4303).
d) 1-(4-Chloro-6-ethyl-2-methyl-pyridin-3-yl)-ethanon wird analog zu Zwischenprodukt 2b, Beispiel 30, nach literaturbekanntem Verfahren hergestellt (J. Heterocyclic Chem., 18, 1981, 603).
e) 1-[4-(4-Bromo-benzylamino)-2-ethyl-6-methyl-pyridin-5-yl]-ethanon wird nach der in Beispiel 30 beschriebenen Vorschrift (Zwischenprodukt 2c), ausgehend von 4-Chloro-2-ethyl-6-methyl-pyridin und 4-Brom-benzylamin, hergestellt. MS(ES+): 347/349.

2-Ethyl-6-methyl-5-acet-1-yl-4-{4-[1-(3α,7α,12α-trihydroxy-10β,13β-dimethyl-17β-(pentansäure-4-yl)-hexadecahydro-cyclopenta[a]phenanthren-3-yl-)ethin-2-yl]-benzyl}-amino-pyridin wird nach dem allgemeinen Verfahren (s. Beispiel 1) hergestellt. MS(ES+): 700.

## Patentansprüche

1. Verbindungen der Formel I 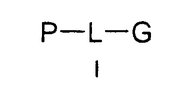 worin bedeuten
G 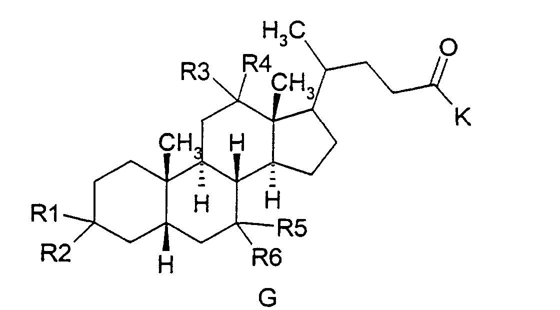
K -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -HN-CHR(9)CO₂H, -OKa, wobei Ka ein Kation bedeutet, wie z.B. ein Alkali- oder Erdalkaliion oder ein quartäres Ammoniumion;
R(7), R(8) unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
R(9) (C₁-C₄)-Alkyl, Benzyl, -CH₂-OH, H₃CSCH₂CH₂-, HO₂CCH₂-, HO₂CCH₂CH₂-;
R(1 ) bis R(6) unabhängig voneinander Wasserstoff, -OR(10), -SR(10), -NR(10)R(13), -OCOR(10), -SCOR(10), -NHCOR(10),-OPO(OR(10))₂, -OSO₂OR(10), - R(10), R(1 ) und R(2), R(3) und R(4), R(5) und R(6) bilden jeweils gemeinsam den Sauerstoff einer Carbonylgruppe, wobei immer genau einer der Reste R(1) bis R(6) die Bedeutung einer Bindung zu L hat;
R(10), R(13) unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
L (C₁-C₁₅)-Alkylen, wobei eine oder mehrere CH₂-Einheiten durch -CH=CH-, - C=C-, -NR(11 )-, -CO-, -O-, -SO₂- oder -S- ersetzt sein können;
R(11) Wasserstoff, (C₁-C₈)-Alkyl, R(12)-CO-, Phenyl, Benzyl;
R(12) Wasserstoff, (C₁-C₈)-Alkyl, Phenyl und Benzyl,wobei der Phenylkern bis zu 3-fach substituiert sein kann mit F, Cl, CF₃, Methyl, Methoxy;
P 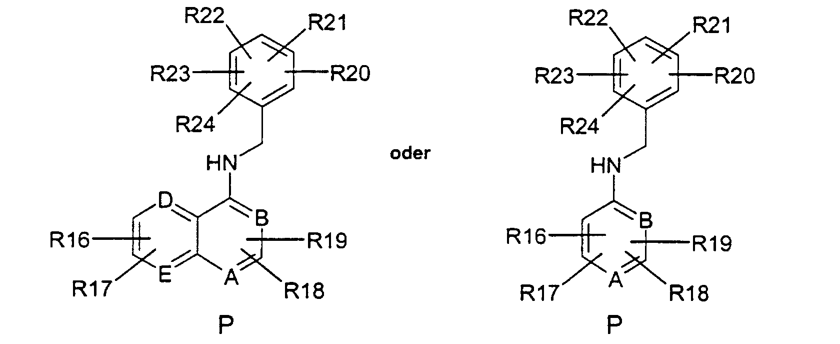
worin bedeuten
A N oder CH;
B N oder CH;
D N oder CH;
E N oder CH;
R(16) bis R(24) unabhängig voneinander Wasserstoff, F, Cl, Br, I, (C₁-C₄)-Alkyl, wobei die Alkylreste ein- oder mehrfach mit Fluor substituiert sein können, CN, NO₂, NR(25)R(26), OR(25), OCOR(25), COR(25), COOR(25), CONR(25)R(26), SO₂R(25), SO₂OR(25), SO₂NR(25)R(26), wobei immer einer der Reste R(16) bis R(24) die Bedeutung einer Bindung zu L hat;
R(25), R(26) unabhängig voneinander Wasserstoff, (C₁-C₄)-Alkyl, wobei die Alkylreste ein- oder mehrfach mit Fluor substituiert sein können, Phenyl und Benzyl;
sowie deren pharmazeutisch verträgliche Salze

2. Verbindungen der Formel I, gemäß Anspruch 1, **dadurch gekennzeichnet, daß** darin bedeuten
G 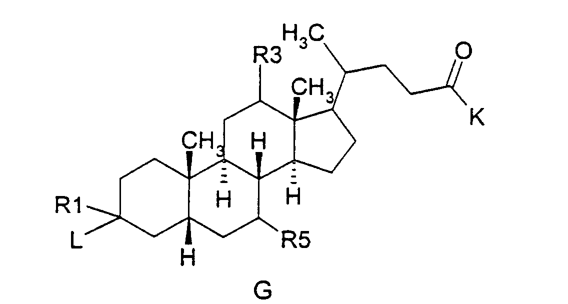
K -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -HN-CHR(9)CO₂H, -OKa, wobei Ka ein Kation bedeutet, wie z.B. ein Alkali- oder Erdalkaliion oder ein quartäres Ammoniumion;
R(7), R(8) unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
R(9) (C₁-C₄)-Alkyl, Benzyl, -CH₂-OH, H₃CSCH₂CH₂-, HO₂CCH₂-, HO₂CCH₂CH₂-;
R(1), R(3), R(5) unabhängig voneinander Wasserstoff, -OR(10), NR(10) R(13), -OCOR(10), -NHCOR(10);
R(10), R(13) unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
L (C₁-C₈)-Alkylen, wobei eine oder mehrere CH₂-Einheiten durch -CH=CH-, -C≡C-, -NR(11 )-, -CO-, -O- oder -SO₂- ersetzt sein können;
R(11) Wasserstoff, (C₁-C₄)-Alkyl, R(12)-CO-, Phenyl, Benzyl;
R(12) Wasserstoff, (C₁-C₄)-Alkyl, Phenyl und Benzyl,wobei der Phenylkern bis zu 3-fach substituiert sein kann mit F, Cl, CF₃, Methyl, Methoxy;
P 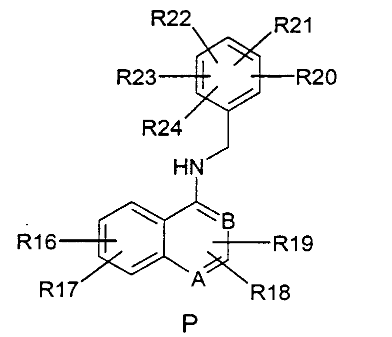
A N oder CH;
B N oder CH;
R(16) bis R(24) unabhängig voneinander Wasserstoff, F, Cl, Br, (C₁-C₄)-Alkyl, wobei die Alkylreste ein- oder mehrfach mit Fluor substituiert sein können, NR(25)R(26), OR(25), OCOR(25), COR(25), COOR(25), CONR(25)R(26), wobei immer einer der Reste R(16) bis R(24) die Bedeutung einer Bindung zu L hat;
R(25), R(26) unabhängig voneinander Wasserstoff, (C₁-C₄)-Alkyl, wobei die Alkylreste ein- oder mehrfach mit Fluor substituiert sein können, Phenyl, Benzyl;
sowie deren pharmazeutisch verträgliche Salze.

3. Verbindungen der Formel I, gemäß Anspruch 1 oder 2, **dadurch gekennzeichnet, daß** darin bedeuten
G 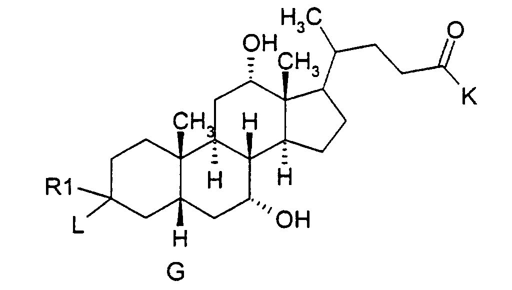
K -OR(7), -NR(7) R(8), -HN-CH₂-CH₂-CO₂H, -HN-CH₂-CH₂-SO₃H, -NH-CH₂-CO₂H, -N(CH₃)CH₂CO₂H, -OKa, wobei Ka ein Kation bedeutet, wie z.B. ein Alkali- oder Erdalkaliion oder ein quartäres Ammoniumion;
R(7), R(8) unabhängig voneinander Wasserstoff, (C₁-C₄)-Alkyl, Phenyl oder Benzyl, wobei der Phenylkern bis zu 3-fach substituiert sein kann mit F, Cl, CF₃, Methyl, Methoxy;
R(1) Wasserstoff, -OH;
L (C₁-C₅)-Alkylen, wobei eine oder mehrere CH₂-Einheiten durch -CH=CH-, -C≡C-, -NR(11 )-, -CO-, -O- oder -SO₂- ersetzt sein können;
R(11) Wasserstoff, (C₁-C₄)-Alkyl, R(12)-CO-, Phenyl, Benzyl;
R(12) Wasserstoff, (C₁-C₄)-Alkyl, Phenyl und Benzyl,wobei der Phenylkern bis zu 3-fach substituiert sein kann mit F, Cl, CF₃, Methyl, Methoxy;
P 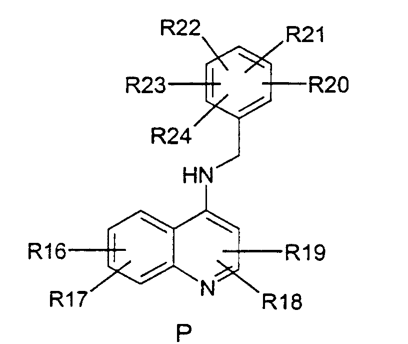
R(16) bis R(24) unabhängig voneinander Wasserstoff, F, Cl, (C₁-C₄)-Alkyl, wobei die Alkylreste ein- oder mehrfach mit Fluor substituiert sein können, NR(25)R(26), OR(25), OCOR(25), COR(25), COOR(25), CONR(25)R(26), wobei immer einer der Reste R(16) bis R(24) die Bedeutung einer Bindung zu L hat;
R(25), R(26) unabhängig voneinander Wasserstoff, (C₁-C₄)-Alkyl, wobei die Alkylreste ein- oder mehrfach mit Fluor substituiert sein können, Phenyl und Benzyl; sowie deren pharmazeutisch verträgliche Salze.

4. Verbindungen der Formel I, gemäß einem oder mehreren der Ansprüche 1 bis 3, **dadurch gekennzeichnet, daß** darin bedeuten
G 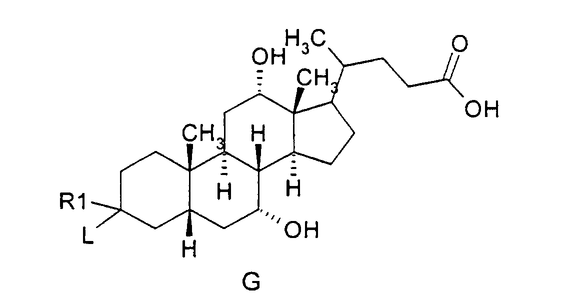
R(1) Wasserstoff, -OH;
L (C₁-C₅)-Alkylen, wobei eine oder mehrere CH₂-Einheiten durch -CH=CH-, -C≡C-, -NR(11 )-, -CO-, -O- oder -SO₂- ersetzt sein können;
P 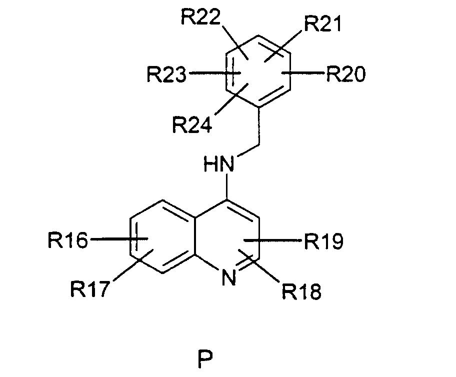
worin bedeutet
R(16) bis R(24) unabhängig voneinander Wasserstoff, F, Cl, (C₁-C₄)-Alkyl, wobei die Alkylreste ein- oder mehrfach mit Fluor substituiert sein können, NR(25)R(26), OR(25), OCOR(25), COR(25), COOR(25), CONR(25)R(26), wobei immer einer der Reste R(16) bis R(24) die Bedeutung einer Bindung zu L hat;
R(25), R(26) unabhängig voneinander Wasserstoff, (C₁-C₄)-Alkyl, wobei die Alkylreste ein- oder mehrfach mit Fluor substituiert sein können, Phenyl und Benzyl;
sowie deren pharmazeutisch verträgliche Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und ein oder mehrere lipidsenkende Wirkstoffe.

7. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

8. Verwendung der Verbindungen gemäß einem oder mehreren der Ansprüche 1 bis 4 zur Herstellung eines Medikaments.

9. Verwendung der Verbindungen gemäß einem oder mehreren der Ansprüche 1 bis 4 zur Herstellung eines Medikaments zur Prophylaxe oder Behandlung von Gallensteinen.

10. Verfahren zur Herstellung der Verbindungen gemäß einem oder mehreren der Ansprüche 1 bis 4, **dadurch gekennzeichnet, daß** man nach folgendem Formelschema 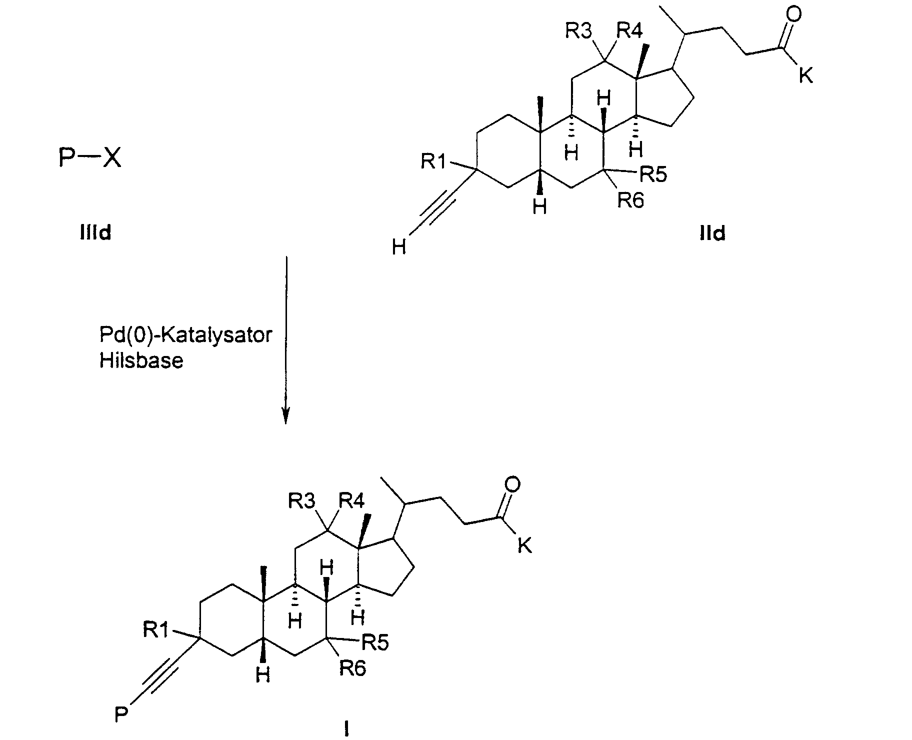 eine Verbindung der Formel IId, worin K, R(1) und R(3) bis R(6) die im Anspruch 1 zu Formel I angegebenen Bedeutungen haben, mit einer Verbindung P-X der Formel IIId, worin P die zu Formel I angegebene Bedeutung hat und X Br oder I darstellt, zur Reaktion bringt.

## Claims

1. A compound of the formula I 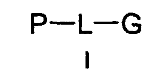 in which
G is 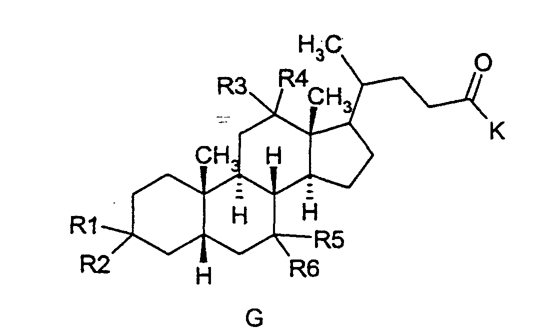
K is -OR(7), -NR(7)R(8), -HN-CH₂-CH₂-CO₂H, -HN-CH₂-CH₂-SO3H, -NH-CH₂-CO₂H, -N(CH₃)CH₂CO₂H, -HN-CHR(9)CO₂H, -OKa, where Ka is a cation such as, for example, an alkali metal or alkaline earth metal ion or a quaternary ammonium ion;
R(7), R(8) are, independently of one another, hydrogen, (C₁-C₄)-alkyl, phenyl or benzyl, it being possible for the phenyl nucleus to be substituted up to 3 times by F, Cl, CF₃, methyl, methoxy;
R(9) is (C₁-C₄)-alkyl, benzyl, -CH₂-OH, H₃CSCH₂CH₂-, HO₂CCH₂-, HO₂CCH₂CH₂-;
R(1) to R(6) are, independently of one another, hydrogen, -OR(10), -SR(10), -NR(10)R(13), -OCOR(10), -SCOR(10), - NHCOR(10), -OPO(OR(10))₂, -OSO₂OR(10), -R(10), R(1) and R(2), R(3) and R(4), R(5) and R(6) in each case form together the oxygen of a carbonyl group, with always exactly one of the radicals R(1) to R(6) having the meaning of a bond to L;
R(10), R(13) are, independently of one another, hydrogen, (C₁-C₄)-alkyl, phenyl or benzyl, it being possible for the phenyl nucleus to be substituted up to 3 times by F, Cl, CF₃, methyl, methoxy;
L is (C₁-C₁₅)-alkylene, it being possible for one or more CH₂ units to be replaced by -CH=CH-, -C≡C-, -NR(11)-, -CO-, -O-, -SO₂- or -S-;
R(11) is hydrogen, (C₁-C₈)-alkyl, R(12)-CO-, phenyl, benzyl;
R(12) is hydrogen, (C₁-C₈)-alkyl, phenyl and benzyl, it being possible for the phenyl nucleus to be substituted up to 3 times by F, Cl, CF₃, methyl, methoxy;
P is 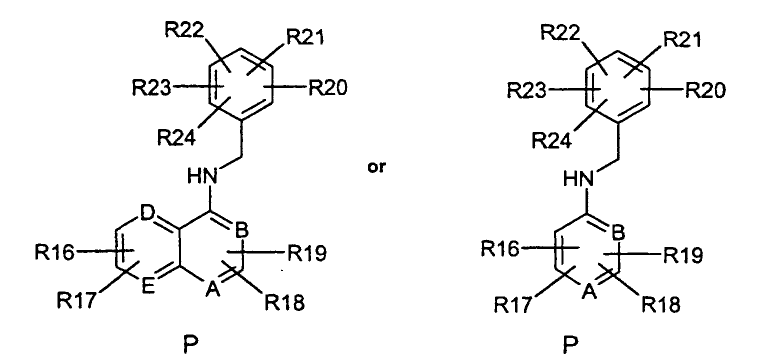
in which
A is N or CH;
B is N or CH;
D is N or CH;
E is N or CH;
R(16) to R(24) are, independently of one another, hydrogen, F, Cl, Br, I, (C₁-C₄)-alkyl, it being possible for the alkyl radicals to be substituted one or more times by fluorine, or are CN, NO₂, NR(25)R(26), OR(25), OCOR(25), COR(25), COOR(25), CONR(25)R(26), SO₂R(25), SO₂OR(25), SO₂NR(25)R(26), with always one of the radicals R(16) to R(24) having the meaning of a bond to L;
R(25), R(26) are, independently of one another, hydrogen, (C₁-C₄)-alkyl, it being possible for the alkyl radicals to be substituted one or more times by fluorine, or are phenyl and benzyl;
and the pharmaceutically acceptable salts thereof.

2. A compound of the formula I as claimed in claim 1, wherein the meanings are
G 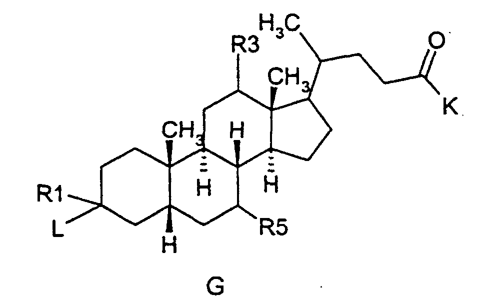
K -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -HN-CHR(9)CO₂H, -OKa, where Ka is a cation such as, for example, an alkali metal or alkaline earth metal ion or a quaternary ammonium ion;
R(7), R(8) independently of one another hydrogen, (C₁-C₄)-alkyl, phenyl or benzyl, it being possible for the phenyl nucleus to be substituted up to 3 times by F, Cl, CF₃, methyl, methoxy;
R(9) (C₁-C₄)-alkyl, benzyl, -CH₂-OH, H₃CSCH₂CH₂-, HO₂CCH₂-, HO₂CCH₂CH₂-;
R(1), R(3), R(5) independently of one another hydrogen, -OR(10), NR(10) R(13), -OCOR(10), -NHCOR(10);
R(10), R(13) independently of one another hydrogen, (C₁-C₄)-alkyl, phenyl or benzyl, it being possible for the phenyl nucleus to be substituted up to 3 times by F, Cl, CF₃, methyl, methoxy;
L (C₁-C₈)-alkylene, it being possible for one or more CH₂ units to be replaced by -CH=CH-, -C≡C-, -NR(11)-, -CO-, -O- or -SO₂-;
R(11) hydrogen, (C₁-C₄)-alkyl, R(12)-CO-, phenyl, benzyl;
R(12) hydrogen, (C₁-C₄)-alkyl, phenyl and benzyl, it being possible for the phenyl nucleus to be substituted up to 3 times by F, Cl, CF₃, methyl, methoxy;
P 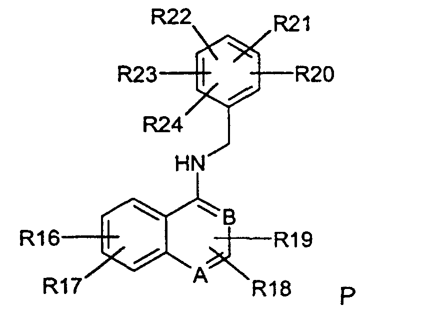
A N or CH;
B N or CH;
R(16) to R(24) independently of one another hydrogen, F, Cl, Br, (C₁-C₄)-alkyl, it being possible for the alkyl radicals to be substituted one or more times by fluorine,
or NR(25)R(26), OR(25), OCOR(25), COR(25), COOR(25), CONR(25)R(26), with always one of the radicals R(16) to R(24) having the meaning of a bond to L;
R(25), R(26) independently of one another hydrogen, (C₁-C₄)-alkyl, it being possible for the alkyl radicals to be substituted one or more times by fluorine,
or phenyl, benzyl;
and the pharmaceutically acceptable salts thereof.

3. A compound of the formula I as claimed in claim 1 or 2, wherein the meanings are
G 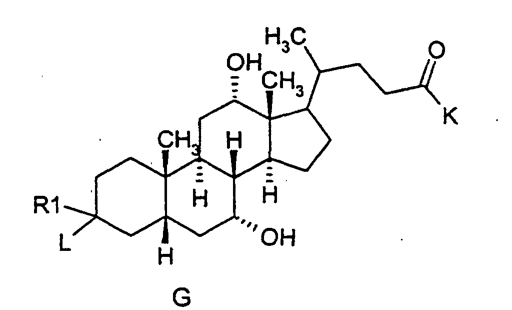
K -OR(7), -NR(7) R(8), -HN-CH₂-CH₂-CO₂H, -HN-CH₂-CH₂-SO₃H, -NH-CH₂-CO₂H, -N(CH₃)CH₂CO₂H, -OKa, where Ka is a cation such as, for example, an alkali metal or alkaline earth metal ion or a quaternary ammonium ion;
R(7), R(8) independently of one another hydrogen, (C₁₋C₄)-alkyl, phenyl or benzyl, it being possible for the phenyl nucleus to be substituted up to 3 times by F, Cl, CF₃, methyl, methoxy;
R(1) hydrogen, -OH;
L (C₁-C₅)-alkylene, it being possible for one or more CH₂ units to be replaced by -CH=CH-, -C≡C-, -NR(11)-, -CO-, -O- or -SO₂-;
R(11) hydrogen, (C₁-C₄)-alkyl, R(12)-CO-, phenyl, benzyl;
R(12) hydrogen, (C₁-C₄)-alkyl, phenyl and benzyl, it being possible for the phenyl nucleus to be substituted up to 3 times by F, Cl, CF₃, methyl, methoxy;
P 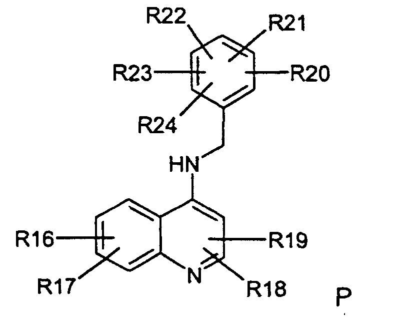
R(16) to R(24) independently of one another hydrogen, F, Cl, (C₁-C₄)-alkyl, it being possible for the alkyl radicals to be substituted one or more times by fluorine,
or NR(25)R(26), OR(25), OCOR(25), COR(25), COOR(25), CONR(25)R(26), with always one of the radicals R(16) to R(24) having the meaning of a bond to L;
R(25), R(26) independently of one another hydrogen, (C₁-C₄)-alkyl, it being possible for the alkyl radicals to be substituted one or more times by fluorine,
or phenyl and benzyl; and the pharmaceutically acceptable salts thereof.

4. A compound of the formula I as claimed in one or more of claims 1 to 3, wherein the meanings are
G 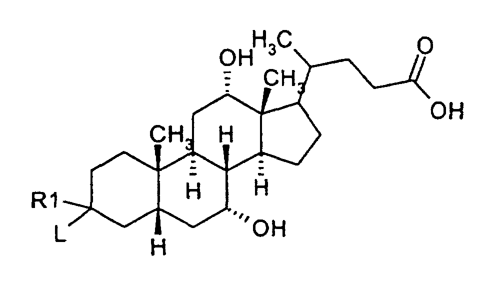
R(1) hydrogen, -OH;
L (C₁-C₅)-alkylene, it being possible for one or more CH₂ units to be replaced by -CH=CH-, -C≡C-, -NR(11)-. -CO-, -O- or -SO₂-;
P 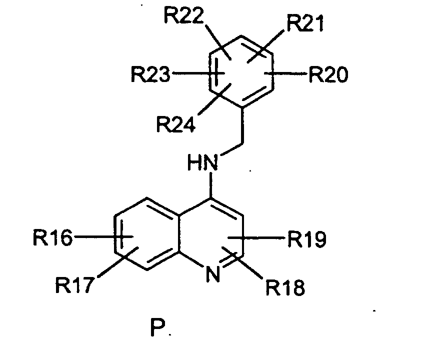
in which
R(16) to R(24) are, independently of one another, hydrogen, F, Cl, (C₁-C₄)-alkyl, it being possible for the alkyl radicals to be substituted one or more times by fluorine,
or NR(25)R(26), OR(25), OCOR(25), COR(25), COOR(25), CONR(25)R(26), with always one of the radicals R(16) to R(24) having the meaning of a bond to L;
R(25), R(26) are, independently of one another, hydrogen, (C₁-C₄)-alkyl, it being possible for the alkyl radicals to be substituted one or more times by fluorine,
or phenyl and benzyl;
and the pharmaceutically acceptable salts thereof.

5. A pharmaceutical comprising one or more of the compounds as claimed in one or more of claims 1 to 4.

6. A pharmaceutical comprising one or more of the compounds as claimed in one or more of claims 1 to 4 and one or more lipid-lowering active ingredients.

7. A process for the production of a pharmaceutical comprising one or more of the compounds as claimed in one or more of claims 1 to 4, which comprises mixing the active ingredient with a pharmaceutically suitable carrier and converting this mixture into a form suitable for administration.

8. The use of the compounds as claimed in one or more of claims 1 to 4 for the production of a medicine.

9. The use of the compounds as claimed in one or more of claims 1 to 4 for the production of a medicine for the prophylaxis or treatment of gallstones.

10. A process for the preparation of the compounds as claimed in one or more of claims 1 to 4, which comprises reacting in accordance with the following formula diagram 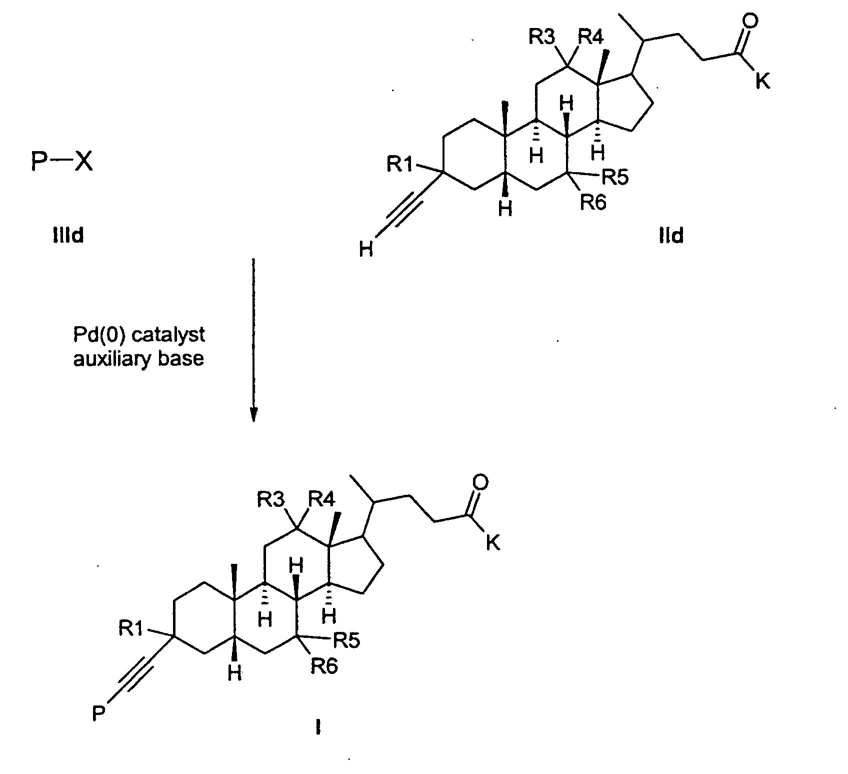 a compound of the formula IId, in which K, R(1) and R(3) to R(6) have the meanings indicated for formula I in claim 1, with a compound P-X of the formula IIId, in which P has the meaning indicated for formula I and X is Br or I.

## Revendications

1. Composés de formule I 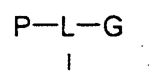 dans laquelle
G représente 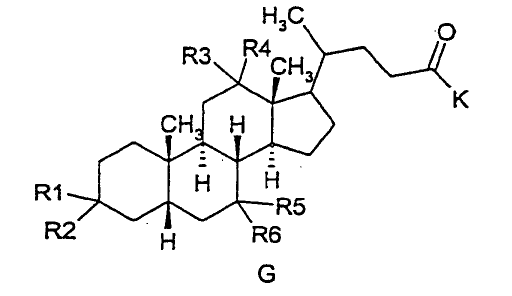
K représente -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -HN-CHR(9)CO₂H, -OKa, où Ka représente un cation tel que, par exemple, un ion de métal alcalin ou alcalino-terreux ou un ion ammonium quaternaire ;
R(7), R(8) représentent, indépendamment l'un de l'autre, un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
R(9) représente un groupe alkyle en C₁ à C₄, un groupe benzyle, -CH₂-OH, H₃CSCH₂CH₂-, HO₂CCH₂-, HO₂CCH₂CH₂- ;
R(1) à R(6) représentent, indépendamment les uns des autres, un atome d'hydrogène, -OR(10), -SR(10), - NR(10)R(13), -OCOR(10), -SCOR(10), - NHCOR(10),-OPO(OR(10))₂, -OSO₂OR(10), -R(10), R(1) et R(2), R(3) et R(4), R(5) et R(6) forment à chaque fois conjointement l'atome d'oxygène d'un groupe carbonyle, l'un des groupes R(1) à R(6) représentant toujours précisément une liaison à L ;
R(10), R(13) représentent, indépendamment l'un de l'autre, un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
L représente un groupe alkylène en C₁ à C₁₅, un ou plusieurs des motifs CH₂ pouvant être substitué par -CH=CH-, -C≡C-, -NR(11)-, -CO-, -O-, -SO₂- ou -S- ;
R(11) représente un atome d'hydrogène, un groupe alkyle en C₁ à C₈, R(12)-CO-, un groupe phényle, benzyle ;
R(12) représente un atome d'hydrogène, un groupe alkyle en C₁ à C₈, un groupe phényle ou benzyle, le noyau phényle pouvant être substitué jusqu'à 3 fois par F, Cl, CF₃, un groupe méthyle, un groupe méthoxy ;
P représente 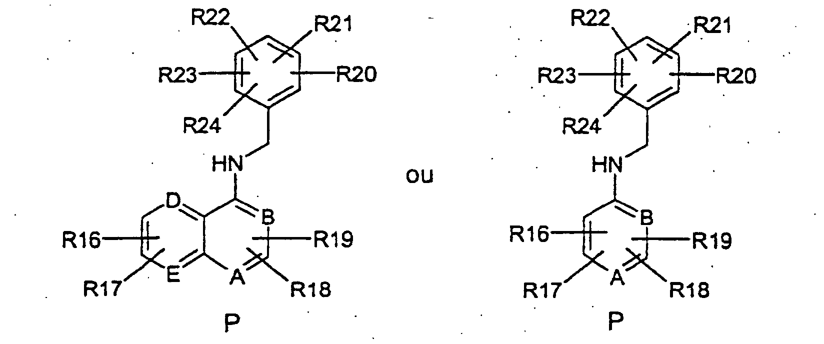
dans laquelle
A représente N ou CH ;
B représente N ou CH ;
D représente N ou CH ;
E représente N ou CH ;
R(16) à R(24) représentent, indépendamment les uns des autres, un atome d'hydrogène, F, Cl, Br, I, un groupe alkyle en C₁ à C₄, les groupes alkyle pouvant être substitués une ou plusieurs fois par un atome de fluor, CN, NO₂, NR(25)R(26), OR(25), OCOR(25), COR(25),. COOR(25), CONR(25)R(26), SO₂R(25), SO₂OR(25), SO₂NR(25)R(26), l'un des groupes R(16) à R(24) représentant toujours une liaison à L ;
R(25), R(26) représentent, indépendamment l'un de l'autre, un atome d'hydrogène, un groupe alkyle en C₁ à C₄, les groupes alkyle pouvant être substitués une ou plusieurs fois par un atome de fluor, un groupe phényle et benzyle ;
ainsi que leurs sels acceptables sur le plan pharmaceutique.

2. Composés de formule I, selon la revendication 1, **caractérisés en ce que**
G représente 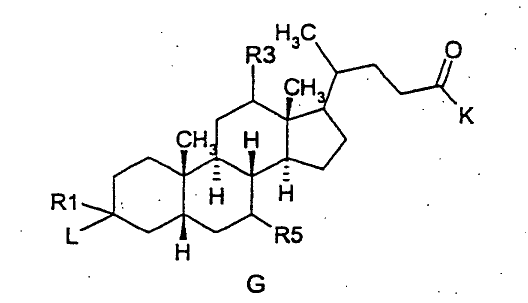
K représente -OR(7), -NR(7)R(8), -HN-CH₂-CH₂-CO₂H, -HN-CH₂-CH₂-SO₃H, -NH-CH₂-CO₂H, -N(CH₃)CH₂CO₂H, - HN-CHR (9) CO₂H, -OKa, où Ka représente un cation tel que, par exemple, un ion de métal alcalin ou alcalino-terreux ou un ion ammonium quaternaire ;
R(7), R(8) représentent, indépendamment l'un de l'autre, un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
R(9) représente un groupe alkyle en C₁ à C₄, un groupe benzyle, -CH₂-OH, H₃CSCH₂CH₂-, HO₂CCH₂-, HO₂CCH₂CH₂- ;
R(1), R(3), R(5) représentent, indépendamment les uns des autres, un atome d'hydrogène, -OR(10), NR(10)R(13), -OCOR(10), -NHCOR(10) ;
R(10), R(13) représentent, indépendamment l'un de l'autre, un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
L représente un groupe alkylène en C₁ à C₈, un ou plusieurs des motifs CH₂ pouvant être substitué par -CH=CH-, -C≡C-, -NR(11)-, -CO-, -O- ou -SO₂- ;
R(11) représente un atome d'hydrogène, un groupe alkyle en C₁ à C₄, R(12)-CO-, un groupe phényle, benzyle ;
R(12) représente un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
P représente 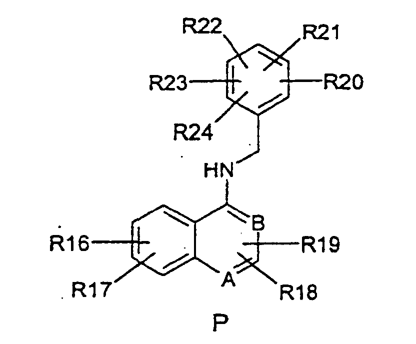
A représente N ou CH ;
B représente N ou CH ;
R(16) à R(24) représentent, indépendamment les uns des autres, un atome d'hydrogène, F, Cl, Br, un groupe alkyle en C₁ à C₄, les groupes alkyle pouvant être substitués une ou plusieurs fois par un atome de fluor, NR(25)R(26), OR(25), OCOR(25), COR(25), COOR(25), CONR(25)R(26), l'un des groupes R(16) à R(24) représentant toujours une liaison à L ;
R(25), R(26) représentent, indépendamment l'un de l'autre, un atome d'hydrogène, un groupe alkyle en C₁ à C₄, les groupes alkyle pouvant être substitués une ou plusieurs fois par un atome de fluor, un groupe phényle, benzyle ;
ainsi que leurs sels acceptables sur le plan pharmaceutique.

3. Composés de formule I, selon la revendication 1 ou 2, **caractérisés en ce que**
G représente 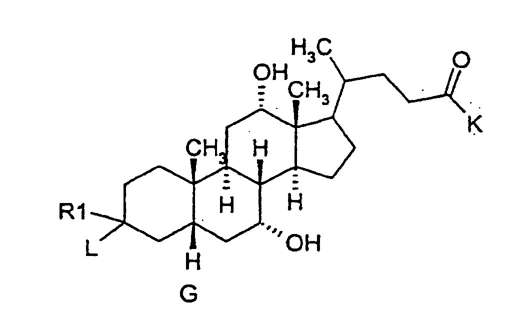
K représente -OR(7), -NR(7)R(8), -HN-CH₂-CH₂-CO₂H, -HN-CH₂-CH₂-SO₃H, -NH-CH₂-CO₂H, -N(CH₃)CH₂CO₂H, -OKa, où Ka représente un cation tel que, par exemple, un ion de métal alcalin ou alcalino-terreux ou un ion ammonium quaternaire ;
R (7), R(8) représentent, indépendamment l'un de l'autre, un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
R(1) représente un atome d'hydrogène, -OH ;
L représente un groupe alkylène en C₁ à C₅, un ou plusieurs des motifs CH₂ pouvant être substitué par -CH=CH-, -C=C-, -NR(11)-, -CO-, -O- ou -SO2- ;
R(11) représente un atome d'hydrogène, un groupe alkyle en C₁ à C₄, R(12)-CO-, un groupe phényle, benzyle ;
R(12) représente un atome d'hydrogène, un groupe alkyle en C₁ à C₄, un groupe phényle ou benzyle, le noyau phényle pouvant être substitué jusqu'à 3 fois par F, Cl, CF₃, un groupe méthyle, un groupe méthoxy ;
P représente 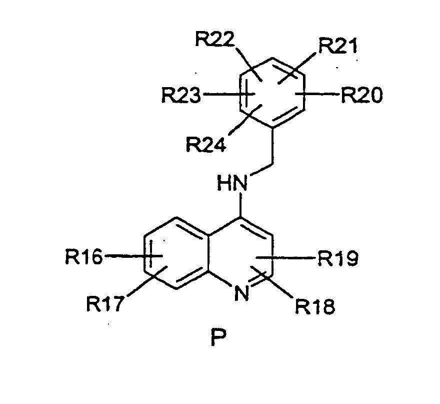
R(16) à R(24) représentent, indépendamment les uns des autres, un atome d'hydrogène, F, Cl, un groupe alkyle en C₁ à C₄, les groupes alkyle pouvant être substitués une ou plusieurs fois par un atome de fluor, NR(25)R(26), OR(25), OCOR(25), COR(25), COOR(25), CONR(25)R(26), l'un des groupes R(16) à R(24) représentant toujours une liaison à L ;
R(25), R(26) représentent, indépendamment l'un de l'autre, un atome d'hydrogène, un groupe alkyle en C₁ à C₄, les groupes alkyle pouvant être substitués une ou plusieurs fois par un atome de fluor, un groupe phényle et benzyle ; ainsi que leurs sels acceptables sur le plan pharmaceutique.

4. Composés de formule I, selon l'une ou plusieurs des revendications 1 à 3, **caractérisés en ce que**
G représente 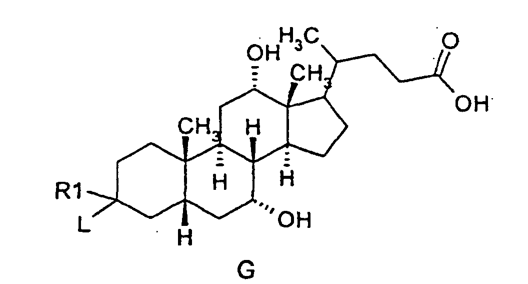
R(1) représente un atome d'hydrogène, -OH ;
L représente un groupe alkylène en C₁ à C₅, un ou plusieurs des motifs CH₂ pouvant être substitué par -CH=CH- , -C≡C-, -NR(11)-, -CO-, -O- ou -SO₂- ;
P représente 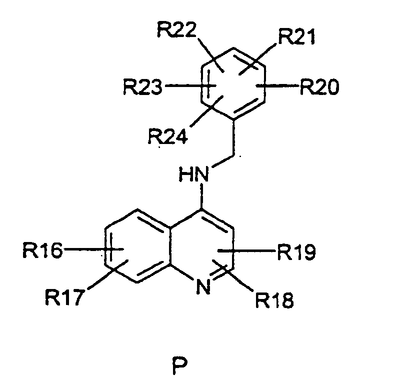
dans laquelle
R(16) à R(24) représentent, indépendamment les uns des autres, un atome d'hydrogène, F, Cl, un groupe alkyle en C₁ à C₄, les groupes alkyle pouvant être substitués une ou plusieurs fois par un atome de fluor, NR(25)R(26), OR(25), OCOR(25), COR(25), COOR(25), CONR(25)R(26), l'un des groupes R(16) à R(24) représentant toujours une liaison à L ;
R(25), R(26) représentent, indépendamment l'un de l'autre, un atome d'hydrogène, un groupe alkyle en C₁ à C₄, les groupes alkyle pouvant être substitués une ou plusieurs fois par un atome de fluor, un groupe phényle et benzyle ;
ainsi que leurs sels acceptables sur le plan pharmaceutique.

5. Médicament contenant un ou plusieurs des composés selon l'une ou plusieurs des revendications 1 à 4.

6. Médicament contenant un ou plusieurs des composés selon l'une ou plusieurs des revendications 1 à 4 et un ou plusieurs principes actifs hypolipémiants.

7. Procédé de préparation d'un médicament contenant un ou plusieurs des composés selon l'une ou plusieurs des revendications 1 à 4, **caractérisé en ce que** l'on mélange le principe actif avec un véhicule approprié sur le plan pharmaceutique et on transforme ce mélange en une forme appropriée pour l'administration.

8. Utilisation des composés selon l'une ou plusieurs des revendications 1 à 4 pour la préparation d'un médicament.

9. Utilisation des composés selon l'une ou plusieurs des revendications 1 à 4 pour la préparation d'un.médicament destiné à la prophylaxie ou au traitement des calculs biliaires.

10. Procédé de préparation des composés selon l'une ou plusieurs des revendications 1 à 4, **caractérisé en ce que** l'on fait réagir, selon le schéma réactionnel suivant 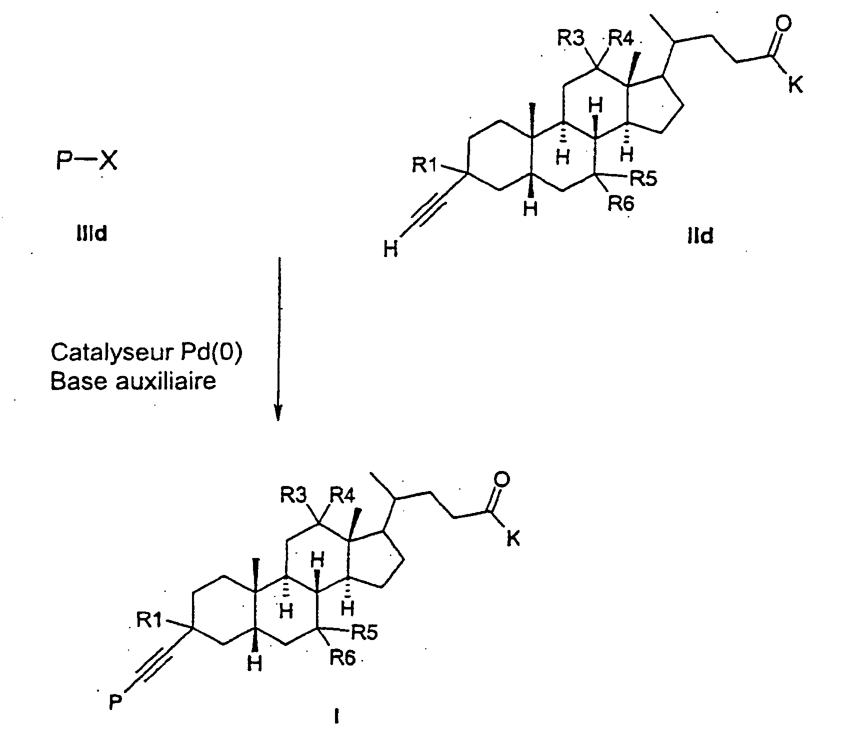 un composé de formule IId, dans laquelle K, R(1) et R(3) à R(6) ont les significations données dans la revendication 1 concernant la formule I, avec un composé P-X de formule IIId, dans laquelle P a la signification donnée pour la formule I et X représente Br ou I.
